# EUROPEAN PATENT SPECIFICATION

(11) **EP 3 047 244 B1**
(45) Date of publication and mention of the grant of the patent: **03.04.2024**
(21) Application number: 14772308.4
(22) Date of filing: 18.09.2014
(51) Int. Cl.: G01J 3/02, G01J 3/46, A45D 44/00, A61B 5/00

(54) **SYSTEMS AND METHODS FOR MEASURING AND CATEGORIZING COLORS AND SPECTRA OF SURFACES**
SYSTEME UND VERFAHREN ZUR MESSUNG UND KATEGORISIERUNG VON FARBEN UND SPEKTREN VON OBERFLÄCHEN
SYSTÈMES ET PROCÉDÉS DESTINÉS À LA MESURE ET À LA CATÉGORISATION DE COULEURS ET DE SPECTRES DE SURFACES

(30) Priority: 19.09.2013 US 201361880157 P
(43) Date of publication of application: 27.07.2016
(73) Proprietor: L'OREAL, 75008 Paris (FR); Spectral Sensors Inc., Marshall, Texas 75670 (US)
(72) Inventor: LEGENDRE, Chloe A., Los Angeles, CA 90094 (US); BALOOCH, Guive, Clark, NJ 07066 (US); LUONGO, Christopher A., Fair Haven, NJ 07704 (US); SLOAN, Walter W., Lake Bluff, IL 60044 (US); PATEL, Paresh, Morgan Hill, CA 95037 (US); LOUDERMILK, Alan R., Marshall, TX 75670 (US)
(74) Representative: Edson, Russell Gregory
(86) International application number: PCT/EP2014/069888
(87) International publication number: WO 2015/040110

(56) References cited:
- WO-A2-2010/067353
- CA-A1- 2 287 687
- US-A- 5 311 293
- US-A1- 2007 076 013
- US-A1- 2007 091 106
- US-B1- 6 603 550
- Anonymous: "Supervised learning - Wikipedia", , 12 October 2012 (2012-10-12), pages 1-8, XP055620640, Retrieved from the Internet: URL:https://en.wikipedia.org/w/index.php?t itle=Supervised_learning&oldid=517423849 [retrieved on 2019-09-10]

## Description

### Field of the Invention

The present invention relates to measuring spectra and other optical characteristics such as colors, translucence, gloss, and other characteristics of objects and materials such as skin, and more particularly to devices and methods for measuring spectra and other optical characteristics of skin or other translucent or opaque objects and for categorizing the measurements into categories relating to products or product formulations to match the measured skin or other object or to transform the skin or other object in a desired manner (e.g., lighten, darken, make more uniform, etc.).

### Background of the Invention

A need has been recognized for devices and methods for measuring spectral, color or other optical characteristics of skin, hair, teeth and other objects, and for predicting or otherwise determining cosmetics (such as foundations), color preparations, restorations or other processes based on such measured optical characteristics.

Attempts have been made to measure skin, teeth, hair and other parts of the body with a variety of different implements with varying degrees of acceptability. One significant problem which must be overcome in the measurement and categorization of color is the inability of current systems to quickly and efficiently categorize large numbers of measurements in a flexible and adaptive system. For example, systems in accordance with the prior art have been used to measure and shade match teeth in a commercially desirous manner.

Other attempts to measure skin, and to classify large numbers of measurements in an adaptive way have been less successful, and the need remains for systems and methods that measure skin, that process data resulting from such measurements to compute color values and/or predict shade or cosmetic products, and that communicate data resulting from such measurements to external computing and/or storage resources.

US 6,603,550 B1 is directed to a skin color measuring device for use with a kit and method to measure skin shades at point-of sale.

US 2007/076013 A1 is directed to a method and apparatus to automatically generate a customized color palette, wherein data corresponding to several physical attributes of a living subject is received and an analysis is conducted which uniquely corresponds to each attribute and associates each attribute with at least one property such as a color family.

CA 2,287,687 A1 is directed to a method and device for the generation of data for the diagnosis of the degree of injury to a patient's skin tissue.

US 5,311,293 A is directed to a method and device for selecting personal compatible colors wherein skin coloration categories are identified based upon blue and yellow undertones of the skin using a color measuring device, developing the Hunter b value, and utilizing that value alone to determine which of several categories of skin coloration a subject's skin exhibits.

US 2007/091106 A1 is directed to a method for assigning a lexical classifier to characterize a visual attribute corresponding to an image element forming part of an image

### Summary of the Invention

According to the invention there is provided a color categorization machine learning method and a color categorization system as defined in the claims.

Based on the prior art, systems and methods for measuring optical properties are provided that enable spectral measurements of skin, hair and other parts of the body. Such systems and methods desirably provide spectral measurements with a handheld device enabling a plurality of desired locations to be measured (e.g., forehead, check and neck areas) conveniently and easily, including in retail sales environments. In accordance with preferred embodiments, the handheld device processes data resulting from the spectral measurements to compute color values (e.g., L*, a*, b* values or L*, C*, h* values), and based on such data and/or such computed values one or more cosmetic products (e.g., a foundation) may be selected. Such selection preferably is determined based on a prediction algorithm which provides a prediction of a cosmetic product that a human observer would affirm is a good
match or otherwise a suitable selection for the skin being measured. In accordance with some preferred embodiments, the prediction algorithm is based on a classifier model that assesses data obtained from clinical studies for selection of the cosmetic products. The prediction algorithm adapts based on the collection of additional data of the device as used in the field or commercial setting. Still preferably, the device operates in accordance with a user interface that guides users (e.g., unscientifically trained users) to make spectral measurements and provide product or other predictions in an intuitive and easy-to-use manner. In accordance with preferred embodiments, the device preferably communicates with an external computer network, which may be by wired (e.g., USB) connection but preferably is wireless (WiFi, Bluetooth^{™}, etc.). In preferred embodiments, the device communicates wirelessly to an external network and also to a companion device such as a smartphone, tablet, notebook computer or other computing device.

Accordingly, it is an object of the present invention to provide systems and methods for measuring spectral or other optical properties of skin, hair or other parts of the body.

It is another object of the present invention to provide systems and methods for communicating data resulting from such measurements, or predictive or other assessments based on such resulting data, via a display integral to the device to communicating via a wired or preferably wireless data connection to an external network.

It is yet another object of the present invention to provide a central processing unit which implements a classifier model for predicting, e.g., foundations or other cosmetic products that match or desirably correspond to measured skin (or hair products in the case of measured hair, etc.).

It further is an object of the present invention to provide an intuitive and easy to use interface to guide users, including non-scientifically trained users, in the taking of spectral measurements and the output or communication of measurement data or predictive or other data based thereon.

Finally, it is an object of the present invention to provide systems and methods for processing and communicating such data to a remote centralized network (e.g., the "cloud," for storage, further processing, generation of updated tables for future predictions, etc).

### Brief Description of the Drawings

The above objects and other advantages of the present invention will become more apparent by describing in detail the preferred embodiments of the present invention with reference to the attached drawings in which:
Figs. 1, 2 and 2A illustrate hardware and physical design aspects of certain preferred embodiments of a handheld device in accordance with the system of the present invention;
Fig. 3 is a block diagram illustrating electronic components and/or circuit functions of certain preferred embodiments of a handheld device in accordance with the system of the present invention;
Figs. 4A, 4B and 4C illustrate exemplary calibration, normalization and measurement methods in accordance with certain preferred embodiments of the present invention;
Fig. 5 illustrates a functional schematic diagram of a system in accordance with the present invention;
Figs. 6A, 6B, 6C, 6D, 6E and 6F illustrate principles and aspects of classifier based methods in accordance with certain embodiments of the present invention;
Figs. 7A and 7B illustrate principles and aspects of classifier based methods in accordance with certain preferred embodiments of the present invention; and
Figs. 8A, 8B, 8C, 8D, 8E, 8F, 8G, 8H, 8I, 8J, 8K, 8L, 8M, 8N, 8O, 8P, 8Q, 8R, 8S, 8T, 8U, 8V, 8W, 8X, 8Y, 8Z, 8AA, 8BB, 8CC, 8DD, 8EE, 8FF, 8GG, 8HH, 8II, 8JJ, 8KK, 8LL, 8MM, 8NN, 8OO, 8PP and 8QQ illustrate exemplary screens displayed utilized in accordance with certain preferred embodiments of the present invention.

### Detailed Description of the Preferred Embodiments

The present invention will be described in greater detail with reference to certain preferred and alternative embodiments. As described below, refinements and substitutions of the various embodiments are possible based on the principles and teachings herein.

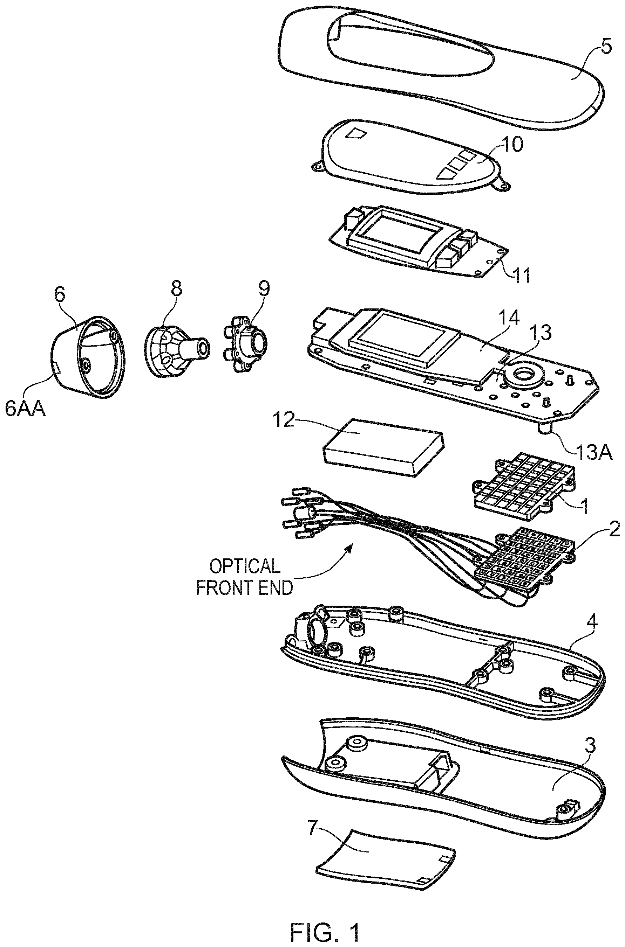

Fig. 1 illustrates an exploded view of a preferred embodiment of a handheld spectrophotometric instrument in accordance with one embodiment of the present invention. As will be appreciated by the skilled reader, however, other optical color measurement devices can also be used in the system of the present invention. A non-limiting example of such an optical color measurement device is a tristimulus colorimeter. In accordance with such preferred embodiments, the instrument is handheld and generally enclosed by handpiece top 5 and handpiece bottom 3. Preferably clear, transparent or translucent LCD cover 10 provides a substantially sealed window for view of a Liquid Crystal Display (LCD) positioned thereunder. In preferred embodiments, a flexible and preferably polymeric LCD housing/buttons implement 11 is provided to surround the LCD display and enable tactile buttons thereunder to be depressed while maintained as secured and preferably dust and moisture prevention sealed enclosure for the instrument. In preferred embodiments, display Printed Circuit Board (PCB) 14 is mechanically and electrically affixed to CPU PCB 13 as illustrated, although in alternative embodiments electronics of the instrument are included on a single PCB or more than two PCBs. What is important is that electronics are provided in the instrument in order to provide functions and operations as more fully described elsewhere herein.

In preferred embodiments, a 10nm visible band spectrophotometer is provided for providing precise spectral measurements, although in other embodiments other spectral resolutions are provided. In accordance with the illustrated embodiment, an optical front end is provided that preferably consists of a center sensor bundle having a terminating end preferably consisting of a metal ferrule positioned in Light Emitting Diode (LED) dome 8. LED dome 8 preferably is a white or near white material such as plastic that provides an optical cavity into which light from a plurality of LEDs is provided. LED dome 8 preferably includes openings into which LEDs are positioned, which are selectively activated under control of a processor included in or on Central Processing Unit (CPU) PCB 13. Also in preferred embodiments, the optical front end includes a plurality of LED tracking fiber bundles, which preferably are held by LED tracking array 9, which fits in a substantially co-axial arrangement with LED dome 8. Handpiece tip 6 fits with handpiece top 5 and handpiece bottom 3 to substantially enclose the internal components as illustrated.

Also as illustrated in Fig. 1, other internal components preferably include battery 12, battery cover 7, and handpiece spine 4. Handpiece spine advantageously provides internal structure to the instrument and in addition eases assembly of the internal components. In preferred embodiments, the spectrophotometer includes up to forty channels, which may include optical sensors mounted on CPU PCB 13, which are surrounded by walls of preferably polymeric "eggcrate" or array fixture 1, which includes cavities or other openings into which preferably bandpass interference filters are positioned. Fiber bundle manifold 2 is provided, into which are securely affixed terminating ends of the main sensor and LED tracking fiber bundles. As will be appreciated by those of skill in the art, the components illustrated in Fig. 1 include details enabling the assembly to be secured such as with screws so that fiber bundle manifold 2, array fixture with filters 1 and CPU PCB 13 are affixed in an appropriate manner. For purposes of explanation additional details such as cabling between CPU PCB 13 and display PCB 14, and connecting wires for powering LEDs that illuminate LED dome 8, are not shown, but will be understood by those of skill in the art.

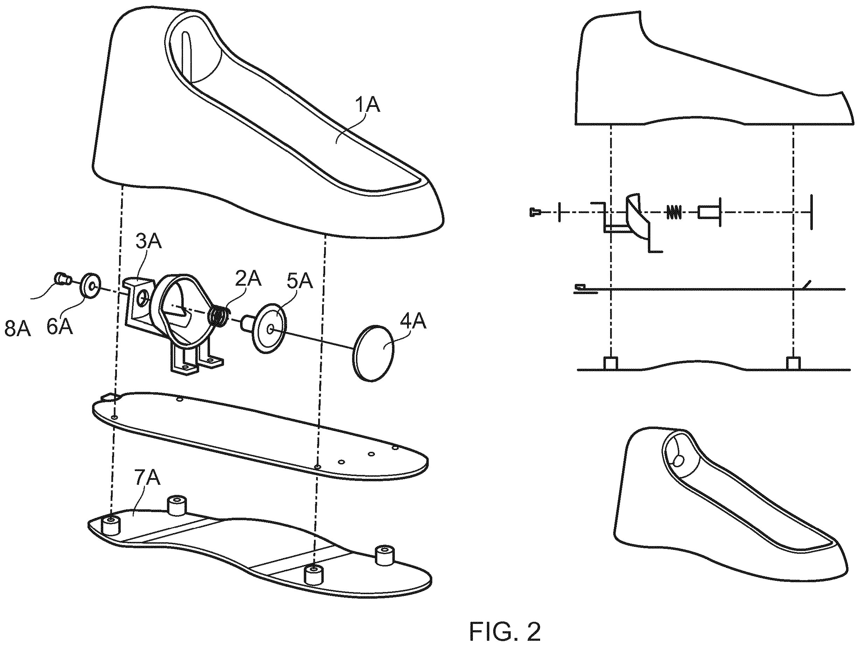

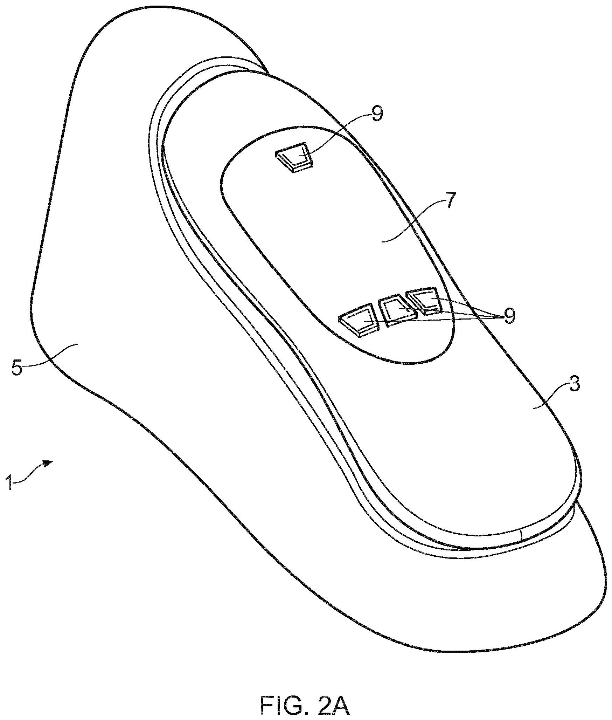

Also as illustrated in Fig. 1, CPU PCB 13 preferably has charging blades or pins 13A which preferably extend through handpiece bottom 3 for electrical engagements with corresponding pins of a charging circuit in a corresponding base unit (see Fig. 2). Also shown in Fig. 1 are magnets 6AA, which preferably are three in number and arranged in and as part of handpiece tip 6. As more fully explained in connection with Fig. 2, magnets 6AA help to bring the end portion of handpiece tip 6 with the surface of an optical reference (see calibration disk 4A of Fig. 2), which in preferred embodiments is used to normalize the instrument prior to at least certain measurements.

Referring to Fig. 2, an exploded view of an exemplary base unit is illustrated, which in preferred embodiments is used to desirably provide a normalization reference implement and a charging system for the instrument. The exemplary base unit including main body 1A, bottom cover 7A, charging board with charging blades or pins that electrically engage charging blades 13A of Fig. 1. A source of electrical power, such as from a transformer, DC power supply, etc., not shown and external to the base unit, provides electrical power to the base unit and provides current for charging the handpiece of the instrument.

As discussed elsewhere herein, in certain preferred embodiments, a calibration or normalization reference is brought into proximity of handpiece tip 6 (see Fig. 1) prior to use of the instrument to measure spectrums. Such a normalization process may desirably accommodate changes in the light output from the LEDs and/or other changes in the optical properties of the measurement system. For precise spectral measurements, it is important that handpiece tip 6 be reliably positioned flush with the calibration or normalization reference. Preferred embodiments provide improved implements and methods for such normalization.

In preferred embodiments, the base unit includes base inside tip 3A, retaining disk 6A, mounting screw 8A, spring 2A and plunger 5A. These elements, and the other elements shown in Fig. 2, provide a movable base for calibration disk 4A, which move to accommodate positioning of handpiece tip 6 inside the base unit and reliably bring handpiece tip 6 into a substantially centered and flush position relative to calibration disk 4A. Also in certain preferred embodiments, calibration disk 4A consists of a white or substantially white coating, such as a fired porcelain or ceramic material, on a metallic substrate. Preferably, the metallic substrate includes or consists of a ferromagnetic material (e.g., steel). In the preferred embodiment, a ceramic material is powder coated and fired to produce a highly inert and durable surface, which is optically stable and may be cleaned. Preferably, an arrangement of magnets 6AA in handpiece tip 6, desirably three magnets in a triangular configuration, are attracted to the magnetic substrate of calibration disk 4A. Thus, in response to a user positioning the handpiece into the base unit, handpiece tip 6 is brought into a flush position relative to calibration disk 4A. Concurrently, charging blades 13A of the handpiece are brought into electrical contact with charging blades of the base unit.

Fig. 2A illustrates an overall view of a preferred embodiment of spectrophotometer system 1 that may be desirably applied to measuring skin, hair and other parts of the human body. Handpiece 3 rests in base unit 5, which generally conforms to the shape of handpiece 3 so that charging contacts of handpiece 3 are brought into electrical contact with corresponding charging contacts in base unit 5. Additionally, the tip of handpiece 3, by the shape of base unit 5 (which may be aided by magnetic-assisted positioning as described elsewhere herein) is brought into a reliable, flush position with a normalization reference included in base unit 5 (all as described in greater detail elsewhere herein). As illustrated, handpiece 3 preferably includes display 7, with buttons 9 preferably positioned in an upper proximate position and in a lower proximate position. While other arrangements of buttons 9 are used in alternative embodiments, in the illustrated embodiment upper button 9 may desirably provide a power on/off (e.g., depress for a predetermined first time interval, such as 2 seconds, to turn on the device, and when in the on state depress for a predetermined second interval of time, such as 5 or 10 seconds, to turn off the device), which also may be used as an activate (e.g., take a measurement) function. Right, left and center lower buttons 9 may be used, for example, to provide a right/left scroll type function, with center lower button 9 providing, for example, a select function. As described elsewhere herein, information displayed on display 7 proximate to particular ones of buttons 9 can indicate to the user the function of the particular button at the point state of operation of the device. In preferred embodiments, different operation modes enable buttons 9 to indicate different functions to the user, and in still other preferred embodiments, the operation of buttons 9 and display 7 may operate in different manners in different points in time based on the contents of Field Programmable Gate Array (FPGA) code utilized in such preferred embodiments.

In accordance with preferred embodiments, the physical shape of the spectrophotometer has a curved overall shape along the length-wise direction such that the planar surface of the tip of the handpiece in which the opening is presented to the portion of the skin being measured is substantially perpendicular to the length-wise direction. The physical shape is arranged so that just the tip of the handpiece is in contact with the portion of the skin being measured.

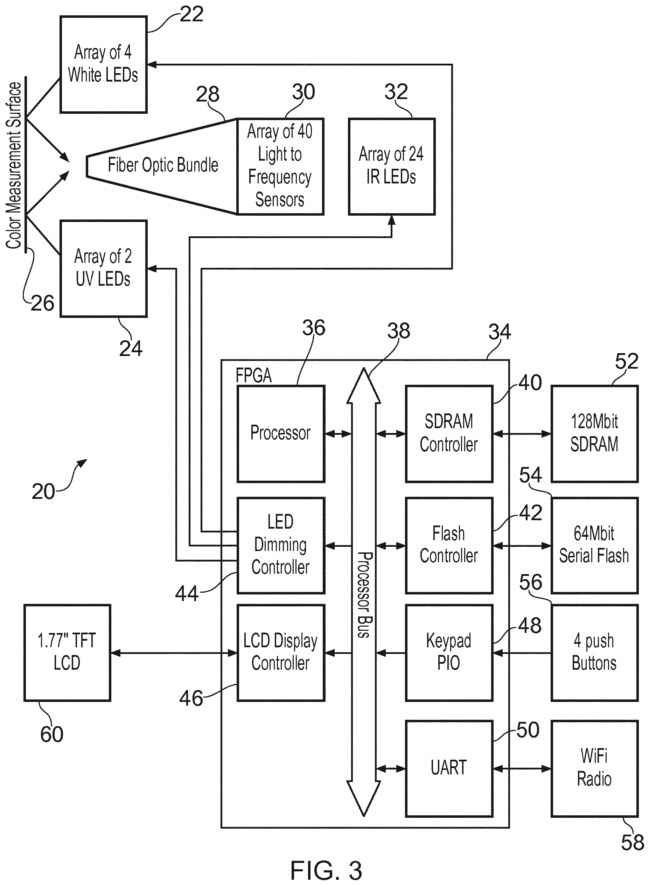

Referring now to Fig. 3, exemplary electronics included in certain preferred embodiments will now be described.

As described elsewhere herein, systems and methods in accordance with certain preferred embodiments of the present invention controllably provide light to a surface or material that is to be optically characterized, which may be skin, hair, teeth, paint, ink, fabric, food, plants, gems, etc., and sense the light returned from the surface material preferably with a plurality of sensors. Preferably, the light source is a broadband light source, such as white LEDs and bulbs of a suitable type. The plurality of sensors preferably include within the sensor light to frequency converters (LFCs) (which may include additional circuitry such as counters and control circuitry so as to output digital values), which receive light after passing through bandpass filters, which preferably consist of interference filters.

As illustrated in Fig. 3, illumination is provided in the exemplary preferred embodiment by LEDs 22 and 24. While other sources of illumination are within the scope of the present invention, LEDs 22 are commercially available white LEDs. As such LEDs typically provide much lower intensity in the shorter wavelengths, in preferred embodiments Ultraviolet (UV) LEDs 24 are optionally provided to increase the illumination intensity in the shorter wavelengths. LEDs 22 and 24 are controlled by FPGA 34, and in particular are controlled by circuitry within FPGA 34 that is logically configured to provide LED dimming controller 44. As will be appreciated by those of skill in the art, LEDs 22 and 24 are controlled to provide illumination of the desired intensity, and the desired duration, to the surface or material to be measured (skin, etc., as denoted by measurement surface 26 in Fig. 3), under control of dimming controller 44.

Based on the characteristics of surface 26, light is received by fiber optic bundle 28 (see also the main sensor bundle of Fig. 1), schematically illustrated in Fig. 3 as a trapezoid but should be understood as bundle of fiber optics. Fiber optic bundle 28 provides a receiver within the optical cavity provided in preferred embodiments, and preferably provides a plurality of outputs that serve to couple light to individual sensors of LFC sensor array 30 via interference filters. While the illustrated embodiment includes 40 individual LFC sensors, in the preferred embodiment 30 of the 40 "channels" are coupled to legs of fiber optic bundle 28, with the respective individual filters being on approximately 10nm centers from, for example, 410 to 700 nm. In the exemplary preferred embodiment, 7 of the remaining 10 channels are used to monitor or track the illumination provided by LEDs 22 and 24, and enabling the spectrum and overall intensity of the illumination to be tracked by processor 36 and software operating thereon. Such multi-channel tracking and monitoring of the illumination allows processor 36 and software to detect illumination drift, and either alerting the operation to re-normalize the instrument, and or in certain embodiments compensate the measurement in view of the illumination drift.

In preferred embodiments, light bias is provided to some or all of the LFC sensors in order to ensure that each sensor is outputting a count at a desired minimum level. In the embodiment illustrated in Fig. 3, bias illumination is controllably provided, under software control via processor 36 and LED dimming controller 44, through infrared (IR) LEDs 32. As will be understood from Fig. 3, IR LEDs may be selectively stepped to increase their output through pulse width modulation so that the desired sensors of LFC array 40 (which may, for example, be only the sensors used for spectral measurements) output pulses at a desired minimum level. In one preferred embodiment, the amount of bias provided by IR LEDs 32 is increased by a predetermined amount, and the sensors measured to determine if the minimum level is achieved, and if not the amount of bias is increased in a stepwise fashion until it is determined that the minimum level is achieved for the desired LFC sensors. In this fashion, a desirable bias level may be achieved in an automatic fashion. In certain preferred embodiments, this automatic bias setting operation is conducted upon instrument startup, and in other embodiments as part of the normalization process before each use. Also, it should be understood that the use of IR LEDs is exemplary, and other illumination sources, such as a halogen or other incandescent type bulb, which desirably is positioned on the backside of CPU PCB (Fig. 1) and provides illumination that may be received by the LFC sensors, for examples, by holes positioned in the PCB underneath the LFC sensors. Such alternative methods of providing bias illumination are within the scope of the present invention.

Also as illustrated in Fig. 3, FPGA 34 preferably includes other elements of a computing system for control of the instrument, including, for example, SDRAM controller 40 (to which may be coupled SDRAM 52), Flash controller 42 (to which may be coupled Flash memory 54, which may be, for example a serial Flash memory), keypad peripheral IO (to which may be coupled push buttons 56, which may be of any desired number such as two, three, four, etc., and which may be capacitive or other types of switches known in the art, but which preferably provide tactile feedback to the user), Universal Asynchronous Receiver/Transmitter (UART) 50 for data communications to external devices, as disclosed in more detail herein, (to which may be coupled a wireless module such as WiFi radio 58, but which could also be Bluetooth^{™} or other standard or custom wired or wireless communication protocols), and LCD displayer controller 46 (to which may be coupled LCD 60 or other suitable display device). Other embodiments include data communication modules such as Universal Serial Bus USB (2.0, 3.0, 1.1, etc.). What is important is that FPGA, which can be an Altera Cyclone III^{™} FPGA, as an example, be configured to include the desired circuit functions for the instrument. While other embodiments include individual circuits such as a CPU, memory controllers, display controller, etc., in preferred embodiments FPGA 34 is used and enables the hardware of the instrument to be readily reconfigured for different applications, updated cosmetic/prediction tables and data, adding product lines to the cosmetic/prediction tables, etc.

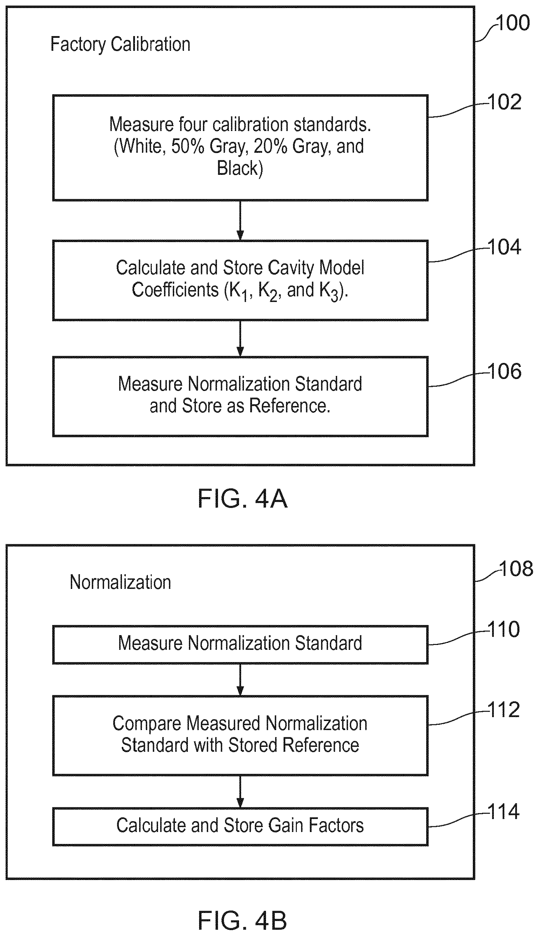

Referring now to Figs. 4A through 4C, exemplary device operations of the handheld spectrophotometer 410 will now be further described.

Operation 100 of Fig. 4A illustrates an exemplary "factory calibration" operation used in preferred embodiments of the present invention. As illustrated in step 102, an individual instrument measures a plurality of calibration standards, preferably at least four in number. The results of such measurements are stored for further processing. As illustrated in step 104, coefficients for linearizing spectral bands are calculated based on the stored data from the measurements in step 102 and stored. Such coefficients in preferred embodiments are Cavity Model coefficients, as described in Sloan W. W., Color Research and Application, "Correction of single-beam sample absorption error in a hemispherical 45°/0° spectrophotometer measurement cavity", 1520-6378, DOI: 10.1002/col.21824. As illustrated in step 106, a calibration/normalization standard (i.e., an optical calibration disk such as disk 4A of Fig. 2), which will accompany the instrument and be used to calibrate or normalize the instrument, is measured and the results stored. Within the device are stored the preferred Cavity Model coefficients, and the reflectance spectrum of the calibration/normalization reference standard that accompanies the instrument. Generation and storage of such data is used in factory calibration in accordance with preferred embodiments. Also, in accordance with preferred embodiments, handpieces and base units (see Figs. 1, 2 and 2A) are serialized (e.g., have serial numbers for the handpiece that matches or other corresponds to the particular base unit with which it was factory calibrated).

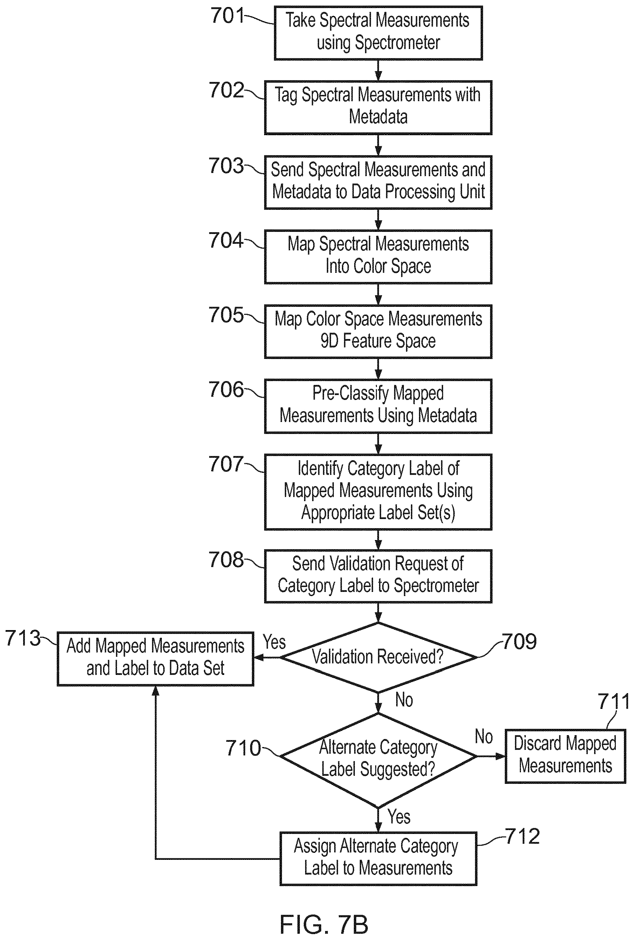

Operation 108 of Fig. 4B illustrates a normalization/calibration operation that is used in preferred embodiments of the present invention. A calibration/normalization reference standard that is measured during factory calibration is positioned in base unit 5 (see calibration disk 4A of Fig. 2). Prior to making measurements, a calibration/normalization operation is performed (see description elsewhere herein, including Fig. 7B and related description) using the same calibration/normalization reference that was used during factory calibration (step 110), and the results from this calibration/normalization measurement are compared with the stored spectral data of the same calibration/normalization reference (step 112). Based on this comparison, gain factors or coefficients are calculated and stored in the instrument (step 114) and used for subsequent measurements with the instrument (until its next calibration/normalization). Such calibration/normalization tends to compensate for changes in the optical performance of the instrument since its factory calibration, including changes in the light source illumination, changes in the optical properties of the cavity, fiber bundles, filters, etc.

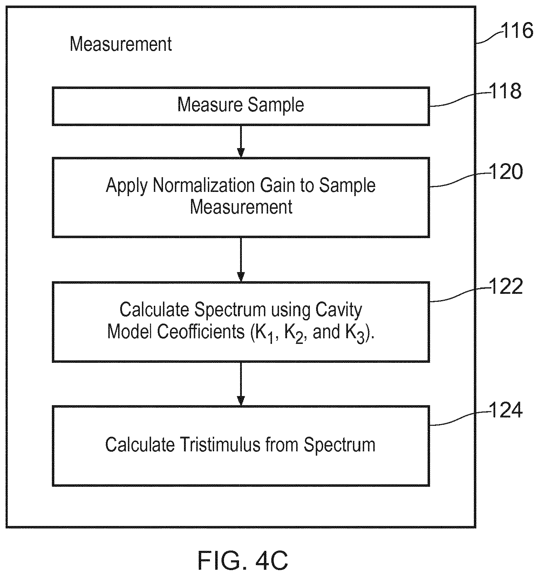

Operation 116 of Fig. 4C illustrates a measurement operation that is used in preferred embodiments of the present invention. At step 118, the sample is measured, which may be skin, hair, or other objects or materials as referenced elsewhere herein. At step 120, the normalization gain factors/coefficients are applied to the spectral data from the measurement at step 118 to produce a normalized spectrum. At step 122, the normalized spectral data from step 120 are adjusted using linearization coefficients, which in the preferred embodiments are Cavity Model coefficients. Step 122 results generally in a normalized, linearized spectrum. Based thereon, at step 124 tristimulus values are calculated. Such values may be used for classification and categorization algorithms, such as described elsewhere herein.

In accordance with preferred embodiments, an optical cavity is presented to the skin or other object or materials under evaluation. The optical cavity receives and reflects preferably broadband light from the illumination source, in preferred embodiments one or a plurality of types of LEDs, with each type of LEDs of one or a plurality of LEDs per type. An opening in the optical cavity allows light to be incident on the surface under evaluation. A portion of this light is received by a receiver, which preferably is a receiver fiber bundle that propagates and couples light to the sensor array via bandpass filters, as described in greater detail elsewhere herein.

A portion of the light returned from the surface under evaluation, however is not directly received by the receiver/sensor bundle, but instead is incident upon a portion of the optical cavity, and which may reflect this light one or more times such that it is re-incident on the surface under evaluation. Herein, this optical effect is referred to as sample substitution error, sample absorption error, etc, which can be quantified and corrected using known methods, such as that disclosed in Sloan W. W., Color Research and Application, "Correction of single-beam sample absorption error in a hemispherical 45°/0° spectrophotometer measurement cavity", 1520-6378, DOI: 10.1002/col.21824.

In a preferred embodiment of the present invention, the handheld spectrophotometer in accordance with the present invention provides further user interface means for inputting measurement-related or other data. The measurement-related or other data can then be incorporated as metadata appended to a particular set of measurements. Non-limiting examples of such metadata can be the age, gender or ethnicity of the subject upon which the measurements are being made. Other examples of metadata that can be used to tag measurement data are a particular range of cosmetics in which categorization of a color is desired, as will be described below. Other metadata can also be used to automatically tag the measurement data. For example, a handheld spectrophotometer or other optical color measurement device for use within the system of the present invention can automatically apply a tag include a time stamp, or locally-generated geographic or retail location information.

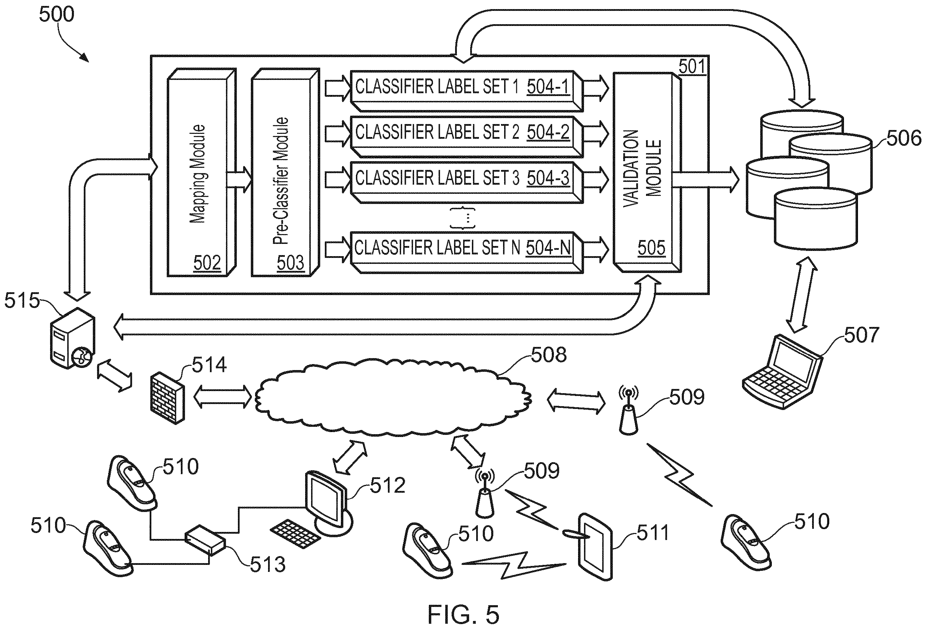

Referring now to Fig. 5, a system in accordance with one embodiment of the present invention will now be described. System 500 comprises a plurality of handheld spectrophotometers 510, situated in a plurality of different geographic locations (which may be, for example, retail or other locations where cosmetic-type products are sold or otherwise provided to users of such products), and a central data processing unit 501 located remotely from each of the handheld spectrophotometers.

As shown in Fig. 5, the base of handheld spectrophotometer 510, and/or the measuring instrument itself, can be connected via a wired connection to a communication hub 513, which itself is connected to a computer 512 connected to a larger network 508, such as the Internet. Furthermore, the base of handheld spectrophotometer 510, and/or the measuring instruments itself, can be wirelessly connected to a tablet computer 511, or the like, via IEEE 802.15.1 (Bluetooth^{™}), IEEE 802.11 (WiFi), or any other suitable wireless technology. Tablet computer 511 can be connected to a wireless access point 509 via IEEE 802.11 (WiFi), or any other suitable wireless technology, which itself can be connected to a larger network 508, such as the Internet. As also shown in Fig. 5, the base of handheld spectrophotometer 510, and/or the measuring instrument itself, can be directly connected to a wireless access point 509 via IEEE 802.11 (WiFi), or any other suitable wireless technology.

The central data processing unit 501 is connected to network 508 via firewall 514 and server 515. Server 515 is arranged to receive measurements and metadata, as well as validation confirmations from handheld spectrophotometers 510, as described below. Server 515 is also configured to send confirmation requests to handled spectrophotometers 510, as well as optional software and firmware updates.

Server 515 is connected to central data processing unit 501. In particular, mapping module 502 of central data processing unit 501 is adapted to receive measurement and metadata information from specific handheld spectrophotometers 510, via server 515. Mapping module maps the measurements received onto a color space, as described below with reference to Fig. 7B. Then the mapped measurements are sent to the pre-classification module 503, which is arranged to use the metadata to select one of several classifier label sets, 504-1, 504-2, 504-3 and 504-N, as also described below with reference to Fig. 7B.

Once the classifiers have categorised the mapped measurements (i.e. once the mapped measurements have been tagged with at least one label of at least one label set), the mapped measurements, along with the measurement metadata and the label tag are passed to the validation module 505 for validation. The validation module 505 is arranged to send a validation request back to one or more of the specific handheld spectrophotometers 510, via server 515. If the categorization is validated, the validation module stores the mapped measurement, along with its tagged label, in memory storage unit 506. The data in memory storage unit 506 can be accessed, analysed and manipulated by way of computer 507. More detailed operation of system 500 will be described below with reference to Fig. 7B.

In machine learning and statistics, "classification" is a methodology for assigning objects to predefined categories. An algorithm that implements classification is known as a "Classifier" and is developed from a set of training data by identifying a set of categories for subpopulations ("Classes") of the data. Each class is defined by one or more quantifiable properties that are referred to as "Explanatory Variables" or "Features." A Classifier is a learning system in that additional data input can be used to adjust the Explanatory Variables and develop new heuristics that improve the accuracy of data classification.

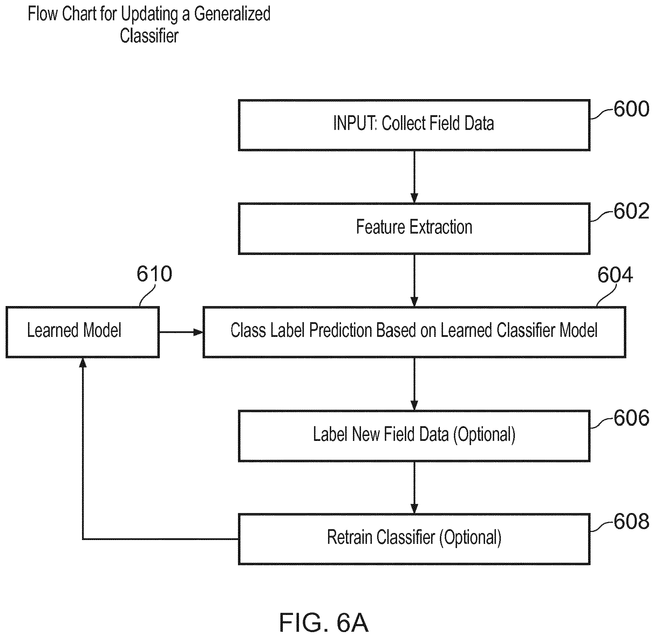

Fig. 6A is a generalized flow chart for developing and updating a Classifier. Data is collected (600) and Features (Explanatory Variables) are extracted from the data (602) and used for class label prediction (604). Many forms and sources of training data are considered to fall within the scope of the present invention. Non-limiting examples of such data are a series of measurements which have been categorized by expert users, or by other types of spectrophotometers or like devices. Additional data may be collected and classified (606), and used to retrain the Classifier (608) by adjusting the Explanatory Variables or adding additional heuristics. The Learned Classifier Model (610) is then used for class label prediction.

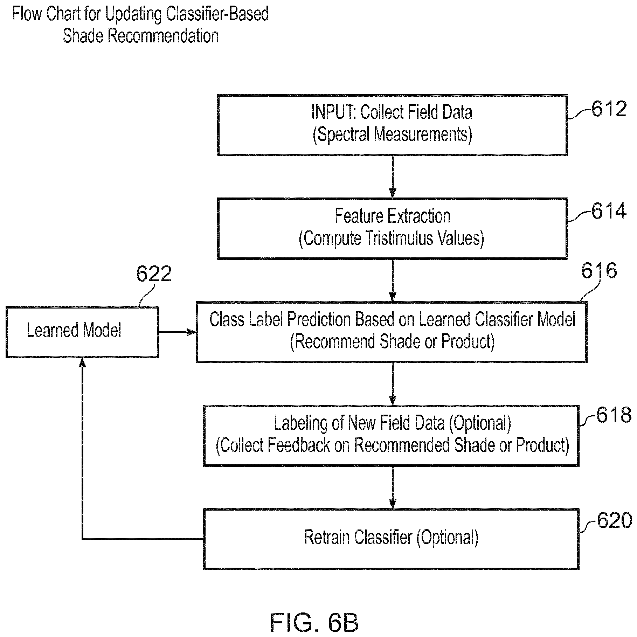

Fig. 6B is a flow chart for developing and updating a Classifier for shade recommendation. Field data is collected in the form of spectral measurements (612) and Features are extracted (614) in the form of the tristimulus calculations for Lightness, Chroma and hue (L*, C* and h*). The Features are used for class label prediction (616). Additional data may be collected and classified (618), and used to retrain the Classifier (620) by adjusting the Explanatory Variables or adding additional heuristics. The Learned Model (622) is then used for class label prediction.

In an alternate preferred embodiment, features are extracted from the field data spectra in the form of other tristimulus calculations (for example CIELAB). These features are then used for class label prediction. In still another alternate preferred embodiment, particular wavelength-dependent spectral reflectance measurements of interest are used directly for the class label prediction. In this embodiment, measurements of interest are optionally determined manually or through an automated method of pertinent feature extraction such as principal component analysis.

In still another preferred embodiment, particular features are extracted from the field data spectra by transforming the spectra into measurements known to be relevant based on the pigment composition(s) of the target measurement substrate. In the case of skin measurement, particular chromophores are known in the literature of skin spectroscopy to be principally responsible for the color appearance of skin: oxyhaemoglobin, deoxyhaemoglobin, and melanin. Reflectance spectra are converted to absorbance spectra, and relative concentrations of these chromophores are determined using formulae from the literature (see: Stamatas, G. N., Zmudzka, B. Z., Kollias, N., & Beer, J. Z. (2008). In vivo measurement of skin erythema and pigmentation: new means of implementation of diffuse reflectance spectroscopy with a commercial instrument. British Journal of Dermatology, 159(3), 683-690). These chromophore concentration variables are used as features for the class label prediction. In an alternate preferred embodiment, combinations of previously described features extracted from spectra are used for class label prediction.

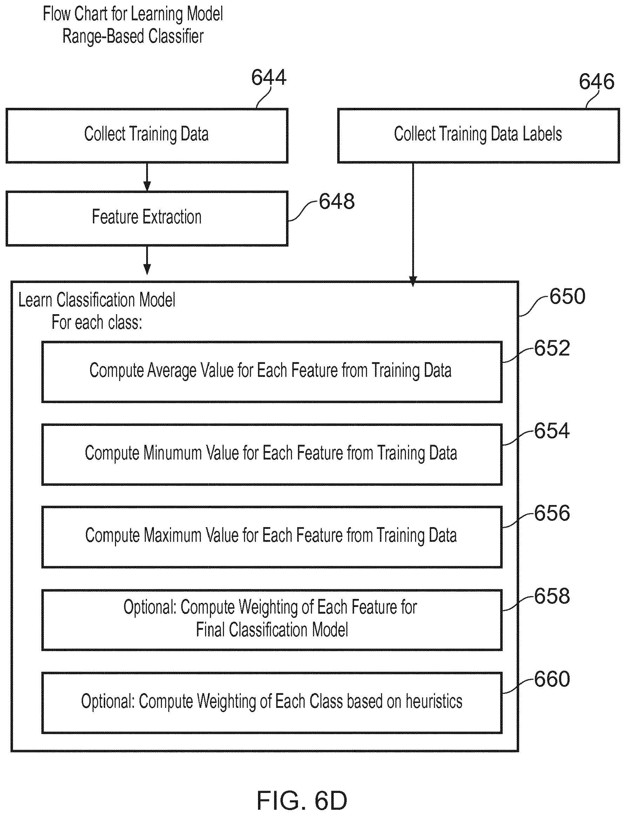

Fig. 6D is a generalized flow chart for a Range-Based Classifier. The Classification Model 650) is developed (Learned) from the Features (648) that are extracted from the training data (644), and the training data labels (646), To build (Learn) the Classification Model for each class, the following calculations are performed for each Feature from the training data:
- Average value (652).
- Minimum value (654)
- Maximum value (656)
- Compute weighting factor from regression (658) (OPTIONAL)
- Compute weighting factor from heuristics (660) (OPTIONAL)

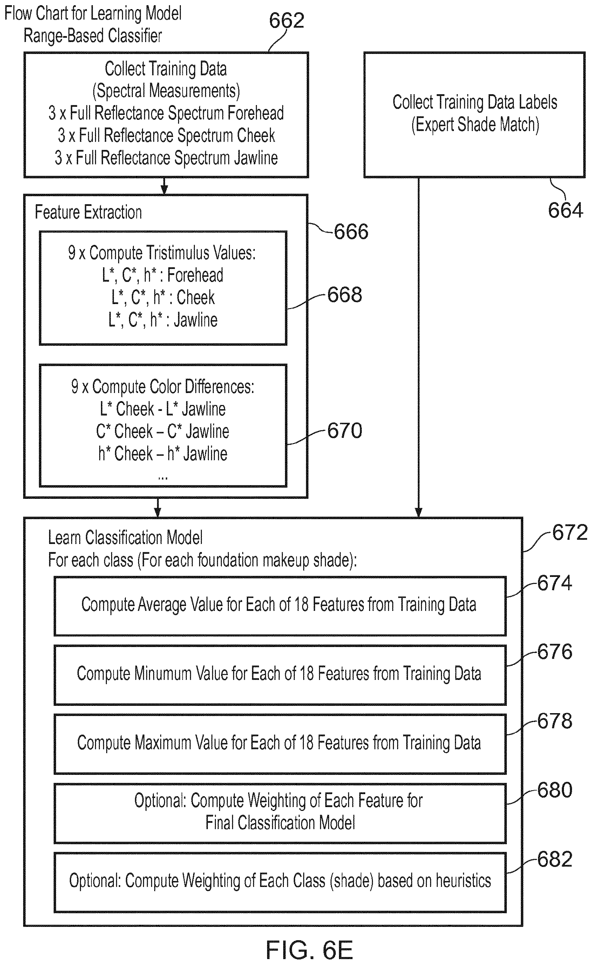

Fig. 6E is a flow chart for the Learning Model for the Range-Based Classifier in this disclosure. Training Data is collected by measuring the full reflectance spectrum of the training subject's forehead, cheek, and jawline (662). Eighteen Features are extracted from the training data (666), as follows:
- Compute tristimulus values (L*, C*, h*) for the Forehead, Cheek, and Jawline (668).
- For each tristimulus value (L*, C*, h*), compute the color difference (670).
   ∘ Cheek - Jawline
   ∘ Cheek - Forehead
   ∘ Jawline - Forehead
   The Classification Model (672) is developed (Learned) for each of the classes (foundation makeup shade) by calculating ranges and averages of each of the Features.
- Compute Average Value for each of the 18 Features from the Training Data (674).
- Compute Minimum Value for each of the 18 Features from the Training Data (676).
- Compute Maximum Value for each of the 18 Features from the Training Data (678).
- (Optional) Compute the weighting of each Feature based on regression (680).
- (Optional) Compute the weighting of each Feature based on heuristics (682).

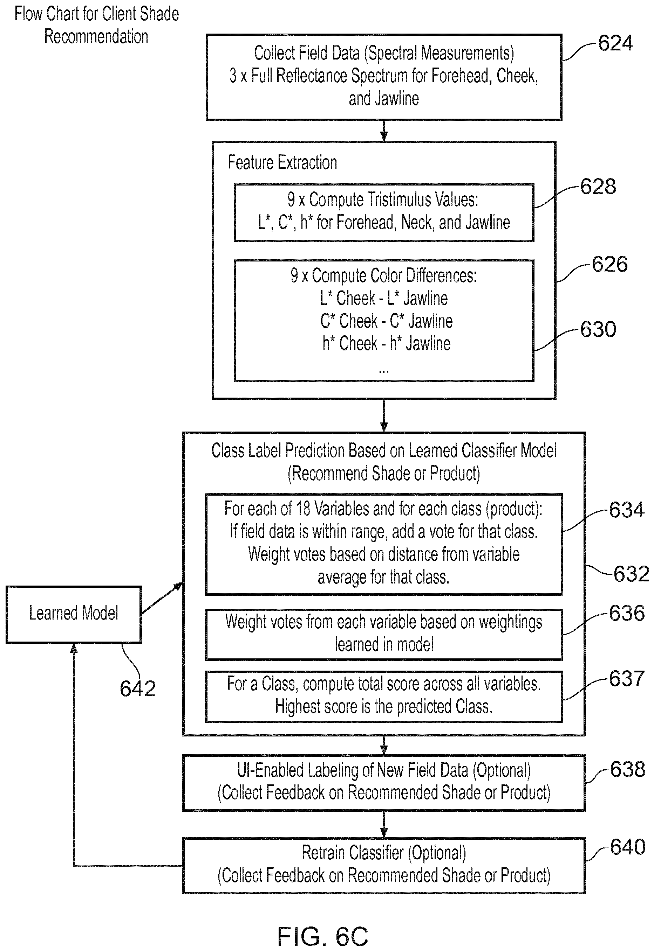

Fig. 6C is a flow chart for client shade recommendation/categorization, based on the Classifier described in 6D. Field Data is collected by measuring the full reflectance spectrum of a subject's Forehead, Cheek, and Jawline (624). Eighteen Features are then extracted from the Field Data (626), as follows:
- Compute tristimulus values (L*, C*, h*) for the Forehead, Cheek, and Jawline (628).
- For each tristimulus value (L*, C*, h*), compute the color difference (630).
   ∘ Cheek - Jawline
   ∘ Cheek - Forehead
   ∘ Jawline - Forehead

The Learned Classification Model (642) is used to assign subjects to classes (foundation makeup shades) by comparing the subject Features with the ranges and averages of each of the classes (632). Each class has its own Minimum, Maximum, and Average for each of the 18 Features.
- For each of the 18 Features, if the Field Data is within range, compute a score for that Feature. The score is dependent on its distance from the average for the class, ranging from one at the average, and decreasing to zero at the minimum or maximum of the range. (634).
- The Learned Model (642) has weighting factors for each of the Features. Multiply the weighting factor for a feature by the score for that feature. Compute the total weighted score for all Features. This is the total score for the class. (636)
- Compare the total scores for each of the classes. The highest score is the predicted class for the measured subject. (637)

If the subject feedback is positive with respect to the shade-match of the predicted class (foundation makeup shade), as described above, label the Field Data with the predicted class. (638) (OPTIONAL)

Retrain the classifier by adding the captured Field Data (640).

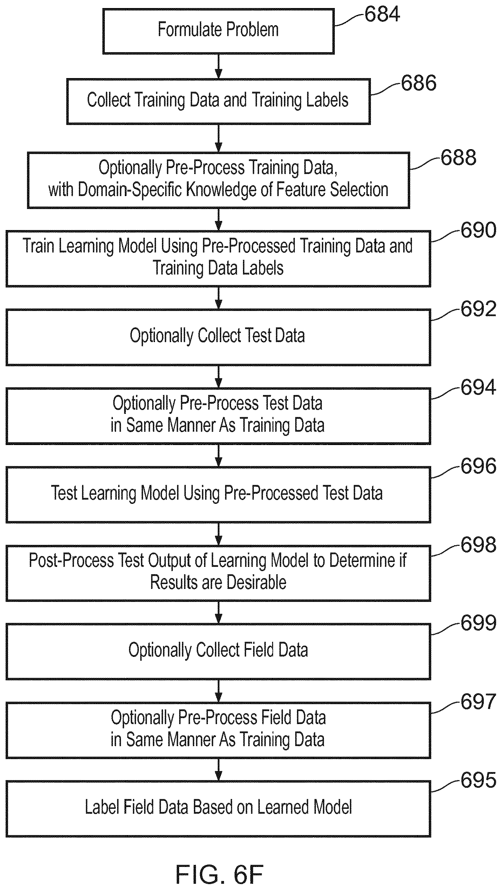

Fig. 6F is a generalized Flow Chart for a linear embodiment of a Classifier Model, showing formulation of the classification problem (684), collection of training data and training labels (686), optionally pre-processing the training data and using that data to train the Learning Model (688, 690), optionally collect and pre-process test data in the same manner as the training data (692, 694), test the Learning Model and determine if the results are desirable (696, 698), and optionally collect and test Field Data in the same manner as the training data (699, 697, and 695). This linear embodiment of a classifier does not call for continual adjustment of the Learning Model as Field Data is acquired and processed.

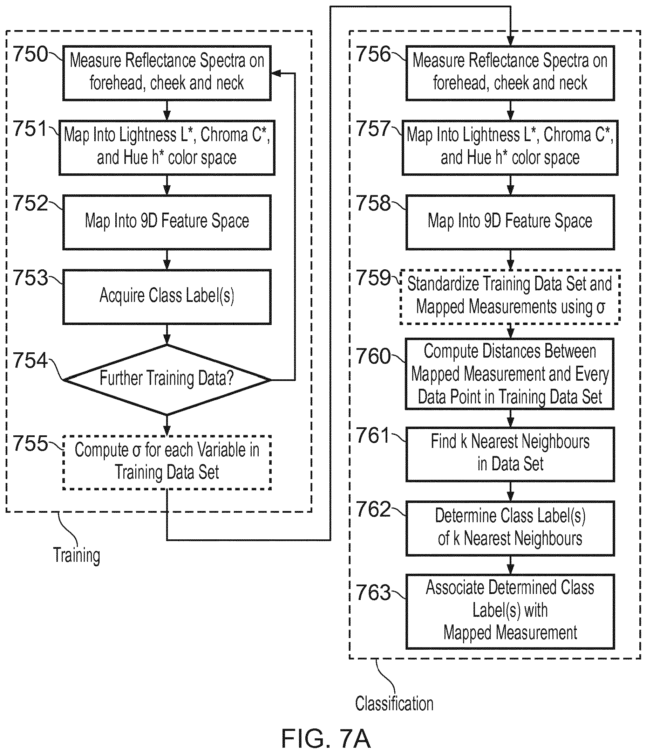

Figs. 7A and 7B are generalized flow charts representing preferred embodiments of a classification model in accordance with the present invention, which classification model is based on a nearest-neighbor based pattern classification algorithm for shade categorization.

The classification model is developed (Learned) from the features that are extracted from the training data and the corresponding training data labels. To build (Learn) the classification model for each class, the following steps (750 to 754, and optionally 755) of the method shown in Fig. 7A are performed for each observation in the training data.

It can be noted that the following model does not assume underlying parameterization (i.e. no probability distribution is assumed for any class), which allows for the use of the algorithm in the case of a small amount of training data when class means and variances are unknowns. In this way, the algorithm is able to mitigate some the difficulty associated with the "cold start problem" in supervised pattern classification (i.e. when there is no initial data to use in a parameterized learning-based model).

At step 750, training data is collected by measuring the full reflectance spectrum of a test subject's forehead, cheek, and jawline, as described above. Then, in step 751, nine features are extracted from the training data by computing tristimulus values (Lightness L*, Chroma C*, and Hue h*) for the forehead, cheek, and jawline. Then, at step 752, these tristimulus values are mapped into a 9-dimensional feature space, with these nine values fully describing each observation (subject) in the training dataset as a 9-dimensional feature vector. As will be appreciated, other forms of mapping spectral measurements onto a color space, along with alternative color spaces, and also be used in conjunction with the present invention.

To complete the training dataset, at least one class label is acquired for each observation in step 753. Multiple classification schemes may be desirable, for instance it may be desired ultimately to classify new observations into one of ten groups, or one of twenty groups, depending on the degree of quantization of feature space for the classification (controlled by the number of shades in a given makeup product line, for example). A class label set is defined herein as a plurality of class labels in the same feature space for a given classification (e.g. for a given product line). Accordingly, multiple class labels (i.e. a single class label from each of a multitude of class label sets) may be acquired for one observation (test subject), for later use in multiple classification routines (for multiple product lines, for example, or for categorization of measurements received from multiple geographic locations).

Additionally, multiple class labels (i.e. multiple class labels selected from a single class label set) may be assigned to one observation even for use in one classification routine. In this situation, more than one categorization may be acceptable for that observation, and this additional information can be used in refining the classification model. At step 754, steps 750 to 753 are repeated until no further training data is available.

For an incoming observation not included in the training dataset, for instance, a new user desiring a shade categorization , field data is collected by measuring the full reflectance spectrum of the subject's forehead, cheek, and jawline at step 756. These measurements are then mapped onto a desired color space in step 757. In a preferred embodiment, the nine features are extracted from the incoming data by computing tristimulus values (Lightness L*, Chroma C*, and Hue h*) for each of the measurement zones. Then, at step 758, the color space measurements are mapped into a 9-dimensional feature space.

For the learning model to work effectively, the measurement zones must be as close as possible to the measurement zones used in generating the training dataset. This can be achieved by, for example, providing a graphical user interface (GUI) on the spectrophotometer in order to guide the user as to measurement placement using an animation of a facial diagram. The measurement vector for the user should be a 9-dimensional feature vector. The training dataset includes a set of 9-D vectors, one set for each observation (subject).

Once the new data is formatted the same way as the class-labeled training data, the classification model to be used is that of the k-Nearest Neighbors (kNN) algorithm. At a high level, this pattern classification algorithm requires a set of class-labeled training data, a new observation formatted identically to the training data, the choice of a distance metric used to compare the distances between observations, and the choice of the number of neighboring observations (k) to be considered in the classification.

In a non-limiting example, k is set to 1, and the distance function is set to the Euclidean distance (L2-norm). The algorithm then proceeds, at step 760, to compute pairwise Euclidean distances between the new mapped measurement and every observation in the dataset. The observation in the dataset closest to the new observation is identified at step 761, along with the class label of this closest observation (step 762). Finally, this class label is returned as the predicted class for the new observation in step 763.

In the kNN algorithm, the variable k is set at a constant integer value to be optimized empirically, representing the number of "neighbors" to be used in the classification. If the value of k is greater than 1, an observation weighting or voting scheme is required for the case of ties. The case of multiple neighbors with different class labels will require some further heuristic for choosing the final best class for a new observation. As more reliable data is collected and incorporated into the training data set, the value of k can be adjusted to improve classification accuracy. Euclidean distance is provided as a preferred distance function, though it is possible to use multiple other distance functions in this algorithm.

In a preferred embodiment, the number of neighbors, k, is set to 1, and the distance function used is the standardized Euclidean distance function in 9-D (as all training data and formatted new observations are 9-D). The training data and incoming new observations are standardized, as will now be described, due to the uneven scaling of the 9 variables.

The standard deviation of each variable of the training data is first computed at step 755. For instance, the standard deviation of the lightness L* of the forehead is computed across all observations of the training set. Prior to classification, all elements of the 9-D vectors of both new observations and the training data are divided by the standard deviation values computed for the corresponding variables, as shown in step 759. In this preferred embodiment, the standard deviation values and the standardized training data are pre-computed for fewer on-line computations. As the training dataset grows in size, exhaustive pairwise distance computations may become too slow, and more complex, alternative data structures designed for such searches, known to those skilled in the art, may be implemented to accelerate classification and reduce the number of distance computations.

In another preferred embodiment, the number of neighbors, k, is set to 1, and the distance function used is a custom-weighted, standardized Euclidean distance function. Depending on the measurements themselves, the weighting (relative importance) of the 9 variables used to measure pairwise distances for each observation differs. For instance, the lightness of the cheek may hold more importance than the lightness of the forehead for users of a certain ethnicity.

In this preferred embodiment, three main classes of measurements are first identified, corresponding to skin tone groups of light, medium, and dark. The light group is defined by the value of lightness (average across Forehead, Cheek, Neck), L* > 54, the medium group is defined as the value of lightness 54 ≥ L* ≥ 40, and the dark group is defined as the value of lightness 40 > L*. Then, for each group, distance function weighting values for each of the 9 variables are set such that the sum of all weights is equal to 1. The optimal weighting values for each skin tone group can be determined through experimentation using the training data. With this approach, pairwise distances between a new observation and training data points will be different and better-suited for predicting classes of new data in each skin tone range. An additional benefit of this approach is the requirement of fewer pairwise distance computations based on a first classification into one of three skin tone sub-groups.

The overall method carried out by the system of the present invention will now be described with reference to Figs. 5 and 7B.

At step 701, a series of measurements are taken using handheld spectrophotometer 510. These measurements include the full reflectance spectrum of the subject's forehead, cheek, and jawline. Once saved to local memory on handheld spectrophotometer 510, at step 702, the measurement data can be tagged with metadata, such as, for example, a time/date stamp, and/or geographic or retail location information associated with the handheld spectrophotometer 510. At step 703, the handheld spectrophotometer 510 sends the measurements, along with any metadata to the central processing unit 501 for categorization. As described above, this is done via network 508, firewall 515 and server 515.

As step 704, the mapping module 502 of central processing unit 501 maps the measurement onto a preferred color space, and then in at step 705, into a 9-dimensional feature space, as described in detail above with reference to Fig. 7A. As will be appreciated by the skilled reader, mapping module 502 can alternatively be implemented in handheld spectrophotometer 510. The mapped measurements are then passed to the pre-classification module 503 to determine which label set(s) (504-1, 504-2, 503-3,...504-N) are to be used to categorise the mapped measurements.

The choice of classifier label set(s) will determine which set(s) of labels will be used to determine the class label(s) in step 762 of Fig. 7A. Non-limiting examples of label sets can include the shades of product lines available in different markets. Each label set can include a different degree of quantization of feature space. Thus, for a first product line, a first label set can be divided into 10 different labels (shades), while for a second product line, a second label set can be divided into ten different labels (shades). Moreover, the feature space of each label set may not be quantized even, and may therefore include "larger" and "smaller" labels covering larger and smaller sections of the feature space.

In essence, the choice of different label sets will typically lead to the application of different labels, thereby resulting in different categorizations. As such, classifiers that use different label sets can be viewed as different classifiers, as they will typically lead to different results.

At step 706, the pre-classifier module 503 uses the metadata received from the handheld spectrophotometer 510 to select which label set(s) is to be used in classifying the mapped measurement. As an example of this, if the metadata includes a geographic and retail location, the pre-classification module 503 can match the geographic and retail location to a particular product line. Once the particular product line is identified, its associated label set can be used for classification. Alternatively, the pre-classification module 503 may determine that in the particular retail establishment in which the measurement was taken (i.e. a given branded shop in a given country or state), more than one product line is available. In such a case, the pre-classification module 503 can select two different label sets, one for each product line.

At step 707, at least one of classifiers 504-1, 504-2, 504-3, ..., 504-N (i.e. classifiers using different label sets) is used to identify at least one category label associated with the mapped measurement. Each of these classifications are carried out as described above, and require access to the data sets stored in the memory storage units 506.

Once the mapped measurements are associated with at least one category label, they are sent, together with their associated at least one category label, to the validation module. At step 708, the validation module sends the at least one category label back to the handheld spectrophotometer 510 that was used to take the measurements and the category (i.e. its related shade) is shown to the user. The user can then be prompted to accept, refuse or ignore the categorization provided.

If the user accepts the categorization, the handheld spectrophotometer sends a validation indication back to the validation module. If, on the other hand, the user refuses the categorization, the handheld spectrophotometer prompts the user to select an alternate categorization. Once selected, the alternate categorization is sent to the validation module for further processing.

At step 709, a determination is made as to whether a validation of the category has been received. This can be performed after a specific period of time. If a validation is received, then the mapped measurement and the category label are added to the relevant data set at step 713. If however no validation is received, a determination is made as to whether an alternate product associated with an alternate category label was suggested. If so, the mapped measurement is assigned the alternate category label in step 712 and both are added to the relevant data set in step 713. Finally, if no validation is received, and no alternate product is suggested, then the mapped measurement can simply be discarded at step 711.

In some embodiments of the present invention, steps 701 to 708 are carried out by the handheld spectrophotometer devices 510 themselves, and the mapped measurements and labels are stored locally for periodic uploading to the central processing unit 501. In these embodiments, classification is performed using the above method, though with a locally stored data set. In such embodiments, the central processing unit receives the validated mapped measurements and labels and adds them to the appropriate data set in memory storage units 506.

Periodically, the central processing unit will can push a data set update to each handheld spectrophotometer device 510, which replaces the locally stored data set with the global or system-wide data set of storage unis 506, in each handheld spectrophotometer device. This global or system-wide accumulation of measurements, combined with the automated pushing of data set updates to the handled devices, allows each spectrophotometer device 510 in the system to benefit from the categorizations effectuated by all (or some) other spectrophotometer devices 510 in the system 500.

As described elsewhere herein, preferred embodiments of the present invention desirably include a display, preferably an LCD, for conveniently providing graphic, textual and numeric information to users to provide an intuitive and powerful user interface. Such a display, in combination with the preferred arrangement of an upper button 9 and preferably three low buttons 9 (see, e.g., Fig. 2A), enable preferred embodiments to provide such an intuitive and powerful user interface.

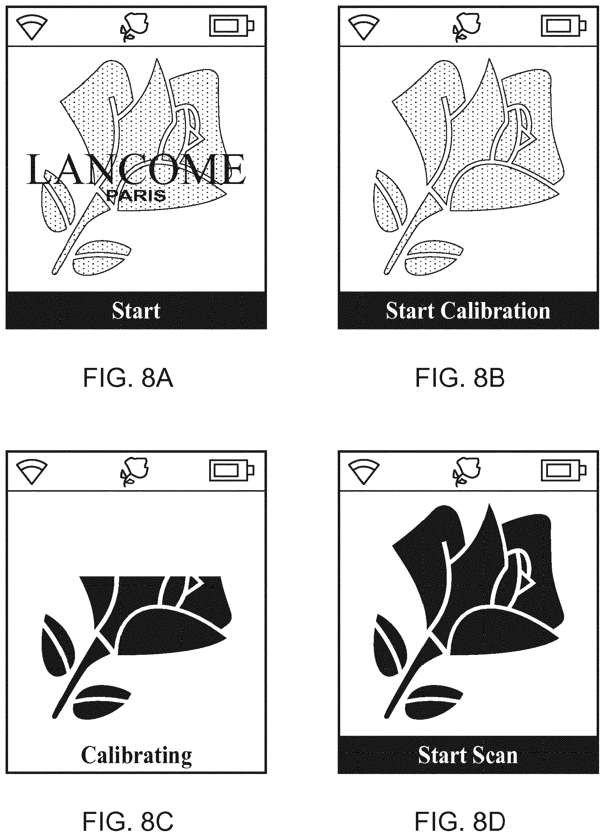

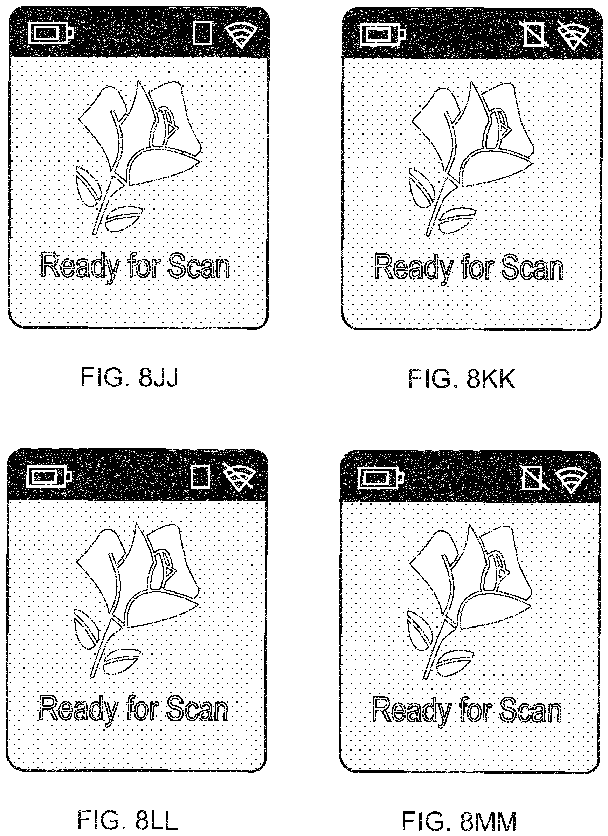

Referring now to Fig. 8A et seq., exemplary screen shots that are displayed will now be described. Based on the exemplary screen shots illustrated and described herein, it will be understood to those of skill in the art that refinements, variations and alterations thereof are within the scope of the present invention.

As illustrated in Fig. 8A, an introductory screen desirably is displayed. This screen may, for example, provide company or product identifiers, and graphics of a desirable type, etc. Preferably, a start indicator is displayed proximal to one of buttons 9 (e.g., lower center button 9), and the start of operation by pressing of the appropriate button 9.

As illustrated in Fig. 8B, in preferred embodiments a calibration start screen is displayed. Preferably, a calibration start indicator is displayed proximal to one of buttons 9 (e.g., lower center button 9), and the start of calibration is initiated by pressing of the appropriate button 9.

As illustrated in Fig. 8C, in preferred embodiments a calibrating screen is displayed, which desirably indicates to the user that the calibration process is underway.

As illustrated in Fig. 8D, in preferred embodiments a start screen is displayed, which indicates that the calibration process has successfully completed. In the event of an unsuccessful calibration, desirably a screen is displayed that prompts the user to initiate (or re-initiate) calibration. In alternate preferred embodiments, the calibration sequence is initiated a plurality of times before the user is prompted to re-initiate calibration, and still alternatively additional messages might be displayed, such as a prompt for power down, technician servicing, cleaning or other maintenance of the calibration reference, etc. Preferably a start scan indicator is displayed proximal to one of buttons 9 (e.g., lower center button 9), and the start of further processing is initiated by pressing of the appropriate button 9.

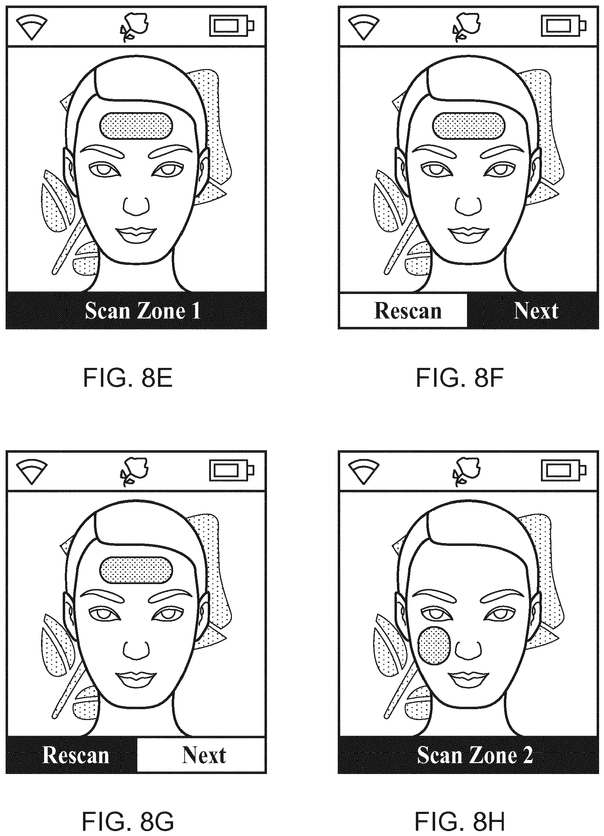

As illustrated in Fig. 8E, in preferred embodiments a scan zone 1 screen is displayed. Preferably an indicator to start a zone 1 scan is displayed proximal to one of buttons 9 (e.g., lower center button 9), and the start of the scan is initiated by pressing of the appropriate button 9. In preferred embodiments, as illustrated a graphic is displayed that guides the user to the approximate location of the zone 1 scan. It should be noted that, in preferred embodiments, a start of scan initiates a plurality of measurements with a single activation, such as three measurements. In certain of such embodiments, an average is taken of the three measurements, spectral band by spectral band. In other embodiments, L*, a*, b*, or L*, C*, h*, values are calculated and averaged. What is important is that in such embodiments a plurality of measurements may be used and averaged to increase data integrity and reliability.

As illustrated in Fig. 8F, in preferred embodiments a screen is displayed that enables the user to rescan zone 1 or proceed to the next step. Preferably the rescan and next indicators are displayed proximal to corresponding one of buttons 9 (e.g., lower left and right buttons 9), and the rescan or progression to the next step is selected by pressing of the appropriate button 9.

Fig. 8G is similar to Fig. 8F but in Fig. 8F the next indicator is highlighted, while in Fig. 8G the rescan indicator is highlighted. In preferred embodiments, once a selection is highlighted, activation of the selected action is initiated by a predetermined one of buttons 9 (e.g., lower center button 9).

As illustrated in Fig. 8H, in preferred embodiments a scan zone 2 screen is displayed. Preferably an indicator to start a zone 2 scan is displayed proximal to one of buttons 9 (e.g., lower center button 9), and the start of the scan is initiated by pressing of the appropriate button 9. In preferred embodiments, as illustrated a graphic is displayed that guides the user to the approximate location of the zone 2 scan.

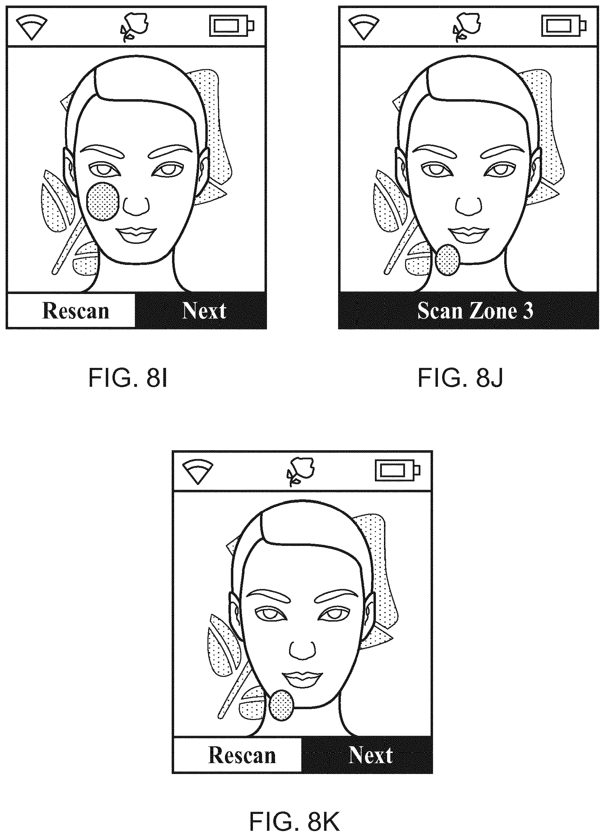

As illustrated in Fig. 8I, in preferred embodiments a screen is displayed that enables the user to rescan zone 2 or proceed to the next step. This screen and associated buttons preferably are displayed and operate similarly to what was described in connection with Figs. 8F and 8G.

As illustrated in Fig. 8J, in preferred embodiments a scan zone 3 screen is displayed. Preferably an indicator to start a zone 3 scan is displayed proximal to one of buttons 9 (e.g., lower center button 9), and the start of the scan is initiated by pressing of the appropriate button 9. In preferred embodiments, as illustrated a graphic is displayed that guides the user to the approximate location of the zone 3 scan.

As illustrated in Fig. 8K, in preferred embodiments a screen is displayed that enables the user to rescan zone 3 or proceed to the next step. This screen and associated buttons preferably are displayed and operate similarly to what was described in connection with Figs. 8F and 8G.

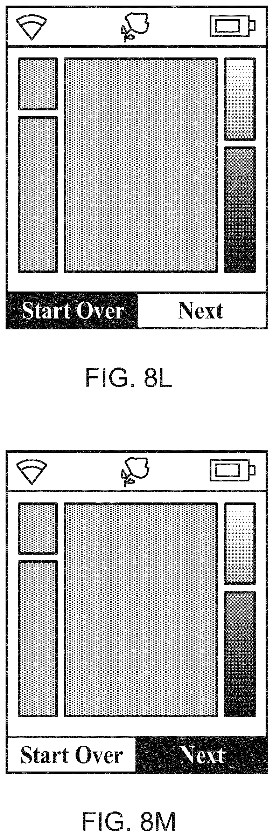

As illustrated in Fig. 8L, in preferred embodiments a screen is displayed that enables the user to start over the scanning process (in alternative preferred embodiments the start over process goes back to the initiate calibration step or alternatively to the scan zone 1 step) or to proceed to the next step. Preferably the start over and next indicators are displayed proximal to corresponding one of buttons 9 (e.g., lower left and right buttons 9), and the start over or progression to the next step is selected by pressing of the appropriate button 9.

Fig. 8M is similar to Fig. 8L but in Fig. 8L the start over indicator is highlighted, while in Fig. 8M the next indicator is highlighted. In preferred embodiments, once a selection is highlighted, activation of the selected action is initiated by a predetermined one of buttons 9 (e.g., lower center button 9).

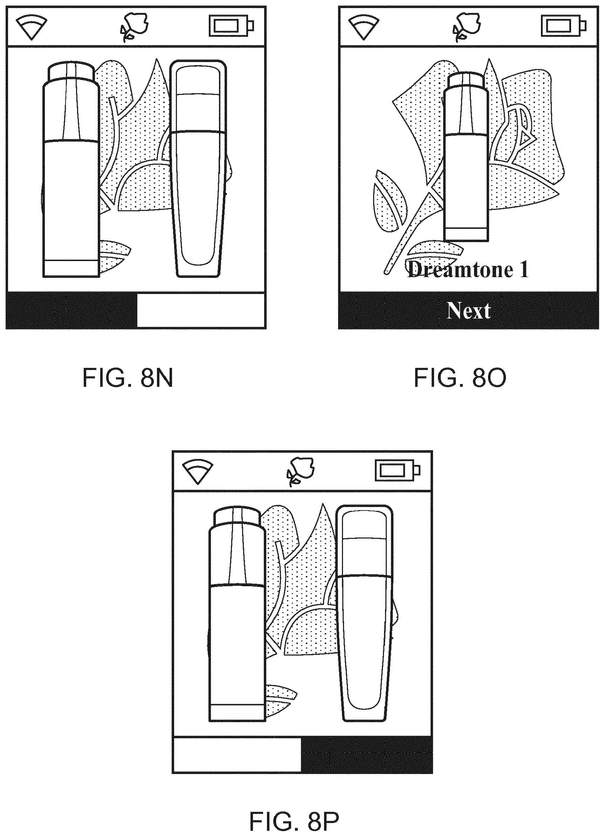

As illustrated in Fig. 8N, in preferred embodiments a first product selection screen is displayed such as is illustrated. Preferably this type of product selection screen enables, for example, the user (such as a beauty advisor) to select a product type for selection or matching based on the spectrophotometric data (or data calculated or otherwise determined therefrom) obtained in previous steps (product selection or matching is described in greater detail elsewhere herein). The exemplary screen of Fig. 8N illustrates a serum-type product of LANCÔME (e.g., DreamTone serum; DreamTone is a trademark of LANCÔME) on the left side, preferably with a graphic of a product bottle or other container or identifier for the serum-type product. The exemplary screen of Fig. 8N illustrates a serum-type product of LANCÔME (e.g., DreamTone serum; DreamTone is a trademark of LANCÔME) on the left side, preferably with a graphic of a product bottle or other container or identifier for the serum-type product. The exemplary screen of Fig. 8N further illustrates a foundation-type product of LANCÔME (e.g., Teint Miracle foundation; Teint Miracle is a trademark of LANCÔME) on the right side, preferably with a graphic of a product bottle or other container or identifier for the foundation-type product. In accordance with preferred embodiments, spectrophotometric data obtained in previous steps may be used to select a target or matching product of a first product type in accordance with a first prediction algorithm, and while such spectrophotometric data may be used to select a target or matching products of a second product type, different from the first product type (e.g., serum v. foundation), in accordance with a second prediction algorithm. Such product line and prediction algorithm may be intuitively and easily selected with graphic image guidance. As will be appreciated by those of skill in the art based on the description herein, such prediction algorithms may be optimized based on the particular characteristics of the product line and product line type.

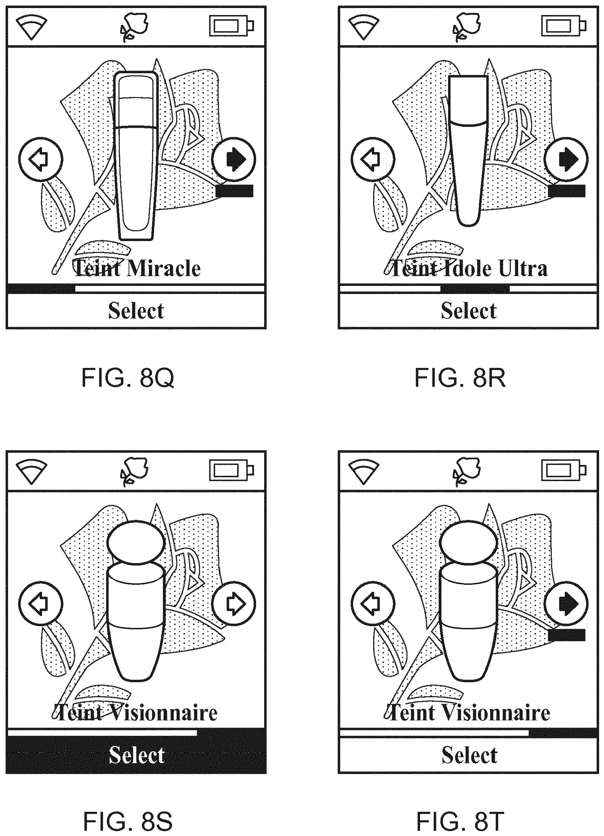

As illustrated in Figs. 8O and 8Q, in preferred embodiments a product type screen is displayed after selection by a user (e.g., beauty advisor). Fig. 8N is similar to Fig. 8P but in Fig. 8N the serum-type product indicator is highlighted, while in Fig. 8P the foundation-type product indicator is highlighted. In preferred embodiments, once a selection is highlighted, activation of the selected action is initiated by a predetermined one of buttons 9 (e.g., lower center button 9).

Referring again to Fig. 8Q, a first foundation-type produce (e.g., Teint Miracle foundation products) is indicated preferably by way of a graphic (exemplary product bottle shown), and an indicator also is provided that shows that the user may also step through other foundation-type products. In Fig. 8R, after pushing a scroll type button, a second foundation-type product (e.g., Teint Idole Ultra; Teint Idole Ultra is a trademark of LANCÔME) is indicated preferably by way of a graphic (exemplary product bottle shown), and one or a plurality of indicators also are provided that shows that the user may step in one or two directions to other foundation-type products (one direction scrolling and two direction scrolling are both within the scope of the present invention). In Fig. 8T, after pushing a scroll type button, a third foundation-type product (e.g., Teint Visionnaire; Teint Visionnaire is a trademark of LANCÔME) is indicated preferably by way of a graphic (exemplary product bottle shown). In preferred embodiments, once a foundation-type selection is selected, activation of a selection is initiated by a predetermined one of buttons 9 (e.g., lower center button 9), and a foundation-type product is selected (see, e.g., Fig. 8S).

As illustrated in Fig. 8S, a foundation-type product may be selected, which either initiates a selection or matching algorithm as described elsewhere herein or initiates the output of a selection or matching algorithm as described elsewhere herein. In preferred embodiments the selection or matching algorithm is initiated automatically for some or all of the product types and lines so that the results may be displayed more promptly after selection of the product line. In other embodiments, the algorithm is initiated after the product type and specific product line is selected.

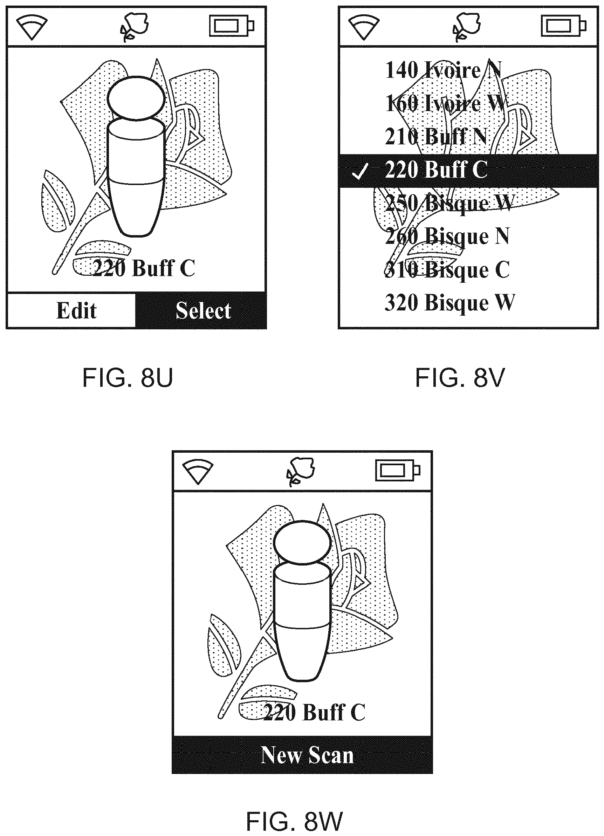

As illustrated in Fig. 8U, in preferred embodiments a selected or matching product is displayed, providing a preferably graphic and alpha numeric display of the selection or match. In Fig. 8U, Teint Visionnaire foundation is the selected product line, and 220 Buff C is the particular foundation product that was determined to be the best or optimum selection or match. It should be understood that best or optimum selection or match may indicate a predicted visual match or a product selection that is predicted to a desired selection based on the end result that the product is to achieve. Also as indicated in Fig. 8U, an indicator preferably is provided such that the user may edit via button push the foundation selection or match, or alternatively may confirm the selection via button push (a confirming selection indicator is shown in Fig. 8U). If the selection or match is confirmed (such as by the beauty or advisor or the end customer applying the foundation and confirming acceptable results), a screen such as is illustrated in Fig. 8W preferably is displayed, which confirms the selection graphically and provides an indicator that a selection may be made to start a new scan with a button push (such as with a new customer, and in such event the sequence may start again at the calibration step or the scan start step, etc).

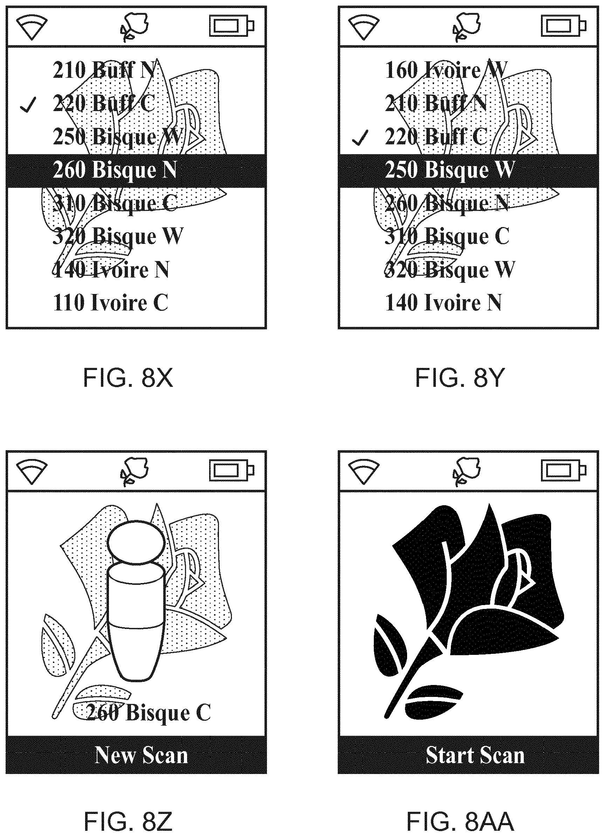

If the user selects via button push to edit the foundation selection, a screen such as is illustrated in Fig. 8V preferably is displayed. While other alternatives are within the scope of the present invention, Fig. 8V provides an indication of the instrument provided selection via check mark, and the current selection by shading. As illustrated in Figs. 8X and 8Y, using button pushes (e.g., left/right lower buttons 9) a scrolling through the products of the selected product line may be obtained. In preferred embodiments, the products of the product line are desirably sorted or ordered by optical characteristics (such as value, or by value and hue, etc.) the scrolling steps through the products in a desired and predetermined order. A button push (e.g., center lower button 9) may be used for the user to enter the alternative selection, which leads to the highlighted selection being displayed as in Fig. 8Z (which is similar to Fig. 8W, as previously described). Figs 8W and 8Z provide an indication of the selected product and the option to start a new scan (which may lead to the display illustrated in Fig. 8AA, or the calibration screen of Fig. 8B, etc.).

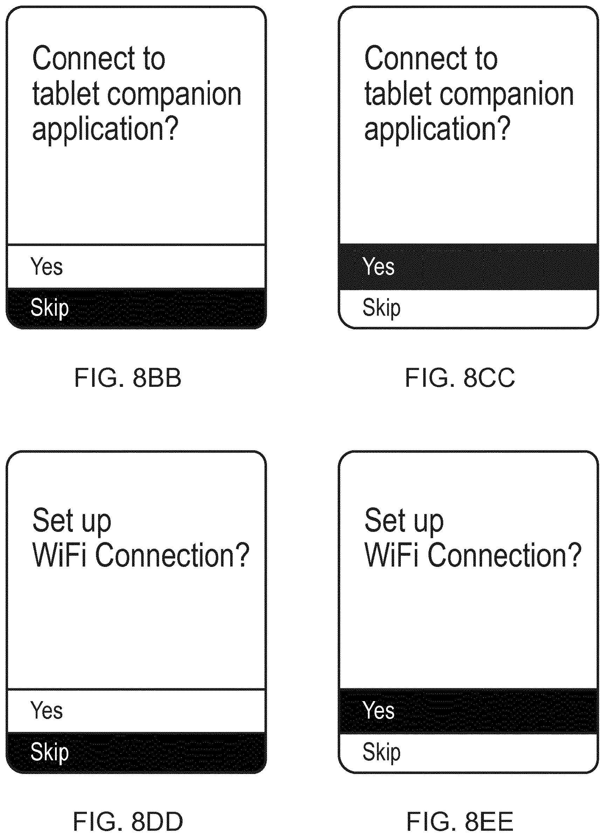

Referring now to Fig. 8BB through Fig. 8EE, exemplary screens are illustrated for connection of the instrument to a companion tablet application and WiFi network. As will be understood from the previous description, buttons 9 may be used to scroll (e.g., left/right lower buttons 9 for up/down, etc.) and make selections (e.g., center lower button 9 for selection). Connection to companion devices (e.g., tablet, computer, smartphone, point of sale terminal, kiosk, etc.) may be made via screens such as in Figs. 8BB and 8CC. Selection of connection to a WiFi network may be made via screens such as in Figs. 8DD and 8EE.

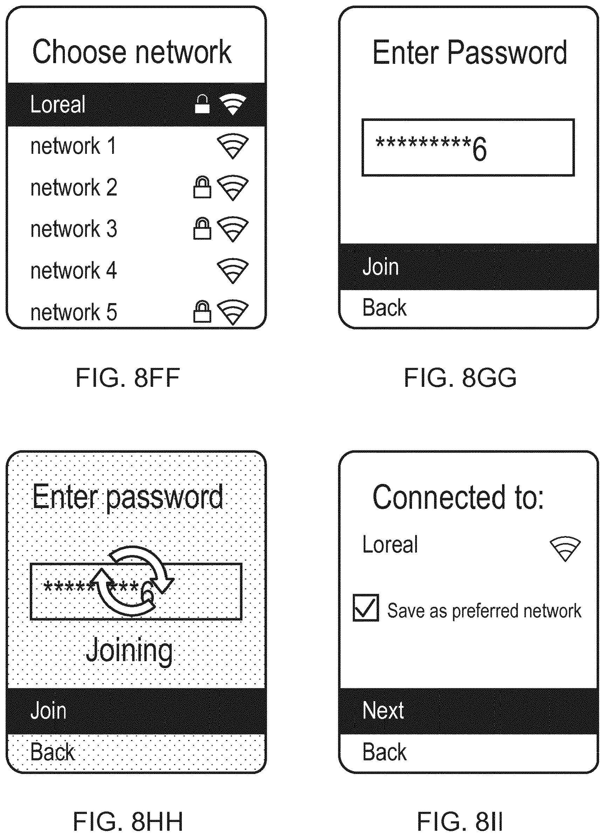

Fig. 8FF illustrates selection of a network from a displayed list of detected networks (network detection may be made a WiFi module as included in preferred embodiments), and Fig. 8GG illustrates password entry for network security (as one example, alpha numeric characters may be displayed, scrolled and selected via buttons 9, as will be understood from description elsewhere herein). Fig. 8HH illustrates an exemplary screen indicating to the user that the instrument is in the process of connecting to the WiFi network. Fig. 8II illustrates an exemplary screen indicating to the user that the instrument is connected to the selected network. Alternates to such screens for connection to companion devices and WiFi networks are within the scope of the present invention.

Figs. 8JJ through 8MM will be understood as providing in preferred embodiments exemplary status icons, such as for battery level and charging status (such as with animation of the battery charge level), connection status for a companion device (such as a tablet or smartphone), and connection status to a WiFi network. Such status icons are within the scope of the present invention.

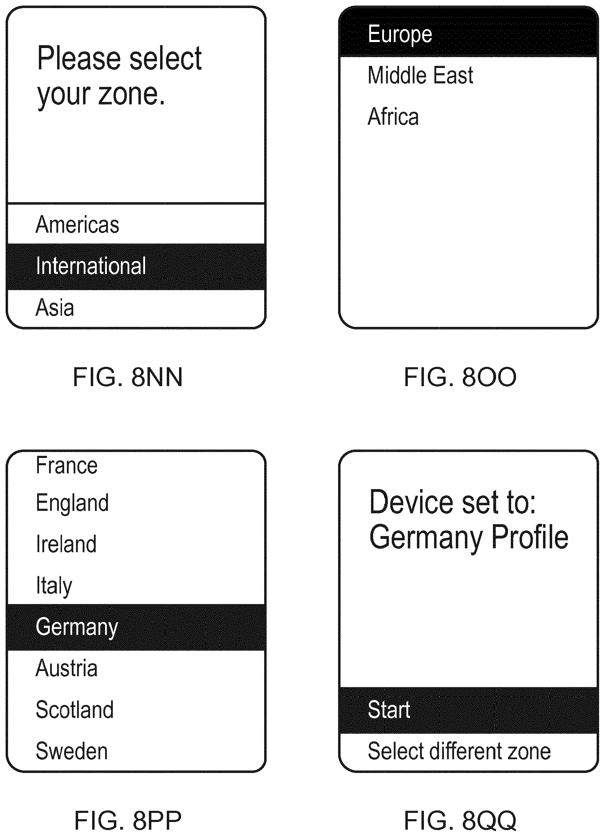

Figs. 8NN through 8QQ will be understood as providing in preferred embodiments exemplary screens for selecting location information, such as geographic region (Fig. 8NN), subregions (Fig. 8OO), and countries (Fig. 8PP). In addition, Fig. 8QQ illustrates setting of a country profile. As will be understood from other description herein, buttons 9 desirably enable the scrolling and selection of such geographic information. Other location information, such as cities, store names, location within stores, etc., also are within the scope of the present invention.

Preferably, and in response to user operation via the exemplary displayed screens, spectrophotometric data is obtained and stored in the instrument, as well as tristimulus color values such as L*, a*, b*, and L*, C*, h*, etc. Data indicative of the instrument selection via a selection/matching algorithm such as described elsewhere herein, and in cases where a selection edit has been made by a user as described above the edited selection, data indicative of the edited selection also are preferably stored in the instrument. In preferred embodiments, the instrument has a persistent or intermittent network connection by which data from the instrument may be connected to a centralized resource, such as a web-connected database. By way of data connection to a centralized web or network resource, which may be made directly via an HTML service provided in the instrument (e.g., functions included within the configuration of FPGA 34 of Fig. 3 preferably includes an HTML service, etc.) or via an intermediate computer, tablet or smartphone, such data preferably is transmitted via the data connection to the centralized resource. Within the scope of the present invention are wireless data connections, such as Bluetooth or WiFi connection, and/or wired data connections, such as USB or LAN.

In accordance with embodiments of the present invention, data is stored within the centralized resource, and in addition subsequent data processing is performed via the centralized resource, which may occur directly with the centralized or remotely via one or more computers or servers having access to data stored in or via the centralized resource, as described above. Such processing, may for example, provide updates to improved selection or matching algorithms and/or tables or other data structures used in the instrument to carry out a prediction algorithm as described elsewhere herein. Also within the scope of the present invention are transmittal to the centralized resource of some or all of the following: information that identifies the instrument (such as via MAC address or other identifier such as device serial number); information that indicates the geographic location of the instrument, such as store or other commercial location of the instrument; information indicative of the software ore firmware version of, for example, the FPGA configuration or other software in the case of non-FPGA embodiments; normalization or calibration data such as obtained via the calibration process initiated via Fig. 8B; information indicative of the normalization or calibration reference used in the instrument, which may be initial normalization data or an identifier for the initial normalization reference; and date and time stamps for the measurement, which may be provided via a real time clock function as a separate module or in the WiFi module, or via a clock that is updated from time to time by network connection (in other embodiments, the time stamp is simply the date and time received by the centralized resource). Such data generation and/or storage in the instrument, and transmission to one or more remote and/or centralized resources (e.g., database, cloud-based data storage or web services) are within the scope of the present invention. In addition, as described elsewhere herein, updates to the configuration data, software, prediction algorithms, prediction data structures also are within the scope of the present invention, and one or more centralized resources desirably provide such update data to instruments as contemplated herein (this may be the same or different centralized resources, but in any event such updates are provided down to the instruments based on data previously transmitted by the instrument up to the centralized resource).

The description and drawings merely illustrate the principles of the invention. For example, the functions of the various elements shown in the figures, including any functional blocks labelled as "modules", may be provided through the use of dedicated hardware as well as hardware capable of executing software in association with appropriate software.

Moreover, explicit use of the term "central data processing unit" should not be construed to refer exclusively to hardware capable of executing software, and may implicitly include, without limitation, digital signal processor (DSP) hardware, network processor, application specific integrated circuit (ASIC), field programmable gate array (FPGA), read only memory (ROM) for storing software, random access memory (RAM), and non-volatile storage. Other hardware, conventional and/or custom, may also be included.

A person of skill in the art would readily recognize that steps of various above-described methods can be performed by programmed computers. Herein, some embodiments are also intended to cover program storage devices, e.g., digital data storage media, which are machine or computer readable and encode non-transitory machine-executable or computer-executable programs of instructions, wherein said instructions perform some or all of the steps of said above-described methods.

The program storage devices may be, e.g., digital memories, magnetic storage media such as a magnetic disks and magnetic tapes, hard drives, or optically readable digital data storage media. The embodiments are also intended to cover computers programmed to perform said steps of the above-described methods. It should be appreciated by those skilled in the art that any block diagrams herein represent conceptual views of illustrative circuitry embodying the principles of the invention.

## Claims

1. A color categorization machine learning method comprising the steps of:
taking (701) color or spectral measurements of a color of a surface of the skin, hair, finger nail, or other part of a body using a color or spectral measurement device;
mapping the color or spectral measurements into a color space;
mapping (705) the color space measurements into a multi-dimensional feature space to generate a multi-dimensional feature vector representative of a plurality of different measured locations of the body;
classifying (707) the mapped color or spectral measurements into a color category using the generated multi-dimensional feature vector and at least one classifier;
generating a validation request (708) for a user of the color or spectral measurement device, the validation request including a request to validate the color category determined in the classifying step; and
adding (713) the mapped color or spectral measurements and color category to training data used by the classifier only if the user of the color or spectral measurement device validates (709) the color category.

2. The color categorization machine learning method of claim 1, wherein the color or spectral measurement device is a spectral measurement device and the color or spectral measurements are spectral measurements.

3. The color categorization machine learning method of claim 2, wherein the color space is a tristimulus, Lightness, Chroma and hue, LCh color space.

4. The color categorization machine learning method of claim 3, wherein:
the surface is the surface of the skin;
the step of taking (701) spectral measurements of a color of a surface using a spectral measurement device includes taking three spectral measurements of the forehead of a subject, three spectral measurements of the cheek of the subject and three spectral measurements of the neck of the subject; and
the step of mapping (705) the spectral measurements into a multi-dimensional feature space includes the step of mapping spectral measurements into a 9-dimensional feature space.

5. The color categorization machine learning method of claim 4, wherein the classifier categorizes using a k-nearest neighbor algorithm in the training data by using at least one of a plurality of category label sets, wherein the distance function of the k-nearest neighbor algorithm is a Euclidian distance function.

6. The color categorization machine learning method of claim 5, wherein k = 1.

7. The color categorization machine learning method of claims 5 to 6, wherein the distance function of the k-nearest neighbor algorithm is a standardized Euclidian distance function, and the training data is standardized by dividing each new mapped measurement and the training data by the standard deviation values computed for the corresponding variables, wherein the distance function of the k-nearest neighbor algorithm is a custom-weighted distance function in which the weighting of the 9 variables used to measure pairwise distances for each measurement differs.

8. The color categorization machine learning method of any of claims 5 to 7, wherein the method further includes the steps of:
collecting (706) metadata relating to the spectral measurements; and
selecting (707), at least in part, based on the metadata, at least one label set to be used in the classifying step, wherein the metadata include at least one of: a time-stamp representing the time at which the measurements were took, details relating to the identity of the subject, the geographic or retail location of the subject.

9. The color categorization machine learning method of any of claim 5 to 8, wherein each category label set includes a plurality of labels relating to color shades of cosmetic products, wherein the category label sets relate to different cosmetic product lines and wherein the category label sets relate to different cosmetic product lines available in the geographic or retail location of the subject.

10. A computer-readable medium comprising instructions for causing a processor, when connected to a color or spectral measurement device, to execute the method steps of any of claims 1 to 9.

11. A color categorization system comprising:
at least one color or spectral measurement device (510) arranged to take color or spectral measurements of a color of a surface of the skin, hair, finger nail, or other part of a body, the at least one color or spectral measurement device being further arranged to communicate the color or spectral measurements to a data processing unit over a data-communication network; and
a central processing unit (501) arranged to:
receive the color or spectral measurements;
map the color or spectral measurements into a color space;
map the color space measurements onto a multi-dimensional feature space to generate a multi-dimensional feature vector representative of a plurality of different measured locations of the body;
classify the mapped color or spectral measurements into a category using the generated multi-dimensional feature vector and at least one classifier;
send an indication of the category to the at least one color or spectral measurement device;
generate a validation request; and
send the validation request to the at least one color or spectral measurement device, the at least one color or spectral measurement device is further arranged to:
display the validation request to the user of the color or spectral measurement device;
accept a validation response from the user of the color or spectral measurement device;
send the validation response to the central processing unit, wherein the central processing unit (501) is further arranged to add the mapped color or spectral measurements and color category to training data used by the classifier if a validation response received from the at least one spectral measurement device (510) is positive.

12. The color categorization system of claim 11, wherein the classifier is arranged to categorize by using a k-nearest neighbor algorithm on the training data and by using at least one of a plurality of category label sets.

13. The color categorization system of any of claims 11 to 12, wherein the system further includes:
metadata collecting means arranged to collet metadata relating to the color or spectral measurements; and
selecting means arranged to select, at least in part, based on the metadata, at least one label set to be used by the classifier, wherein the metadata include at least one of: a time-stamp representing the time at which the measurement were took, details relating to the identity of the subject, the geographic or retail location of the subject.

14. The color categorization system of any of claim 12 to 13, wherein each category label set includes a plurality of labels relating to color shades of cosmetic products, wherein the category label sets relate to different cosmetic product lines, wherein the category label sets relate to different cosmetic product lines available in the geographic or retail location of the subject.

## Patentansprüche

1. Maschinelles Lernverfahren zur Kategorisierung von Farben, umfassend die Schritte:
Vornehmen (701) von Farb- oder Spektralmessungen einer Farbe einer Oberfläche von Haut, Haar, Fingernägeln oder eines anderen Körperteils mit Hilfe eines Farb- oder Spektralmessgeräts;
Abbilden der Farb- oder Spektralmessung in einen Farbraum;
Abbilden (705) der Farbraummessungen in einen mehrdimensionalen Merkmalsraum, um einen mehrdimensionalen Merkmalsvektor zu erzeugen, der für eine Vielzahl von gemessenen Stellen des Körpers repräsentativ ist;
Klassifizieren (707) der abgebildeten Farb- oder Spektralmessungen in eine Farbkategorie unter Verwendung des erzeugten multidimensionalen Merkmals und zumindest eines Klassifikators;
Erzeugen einer Validierungsanfrage (708) für einen Benutzer des Farb- oder Spektralmessgeräts, wobei die Validierungsanfrage eine Anfrage zur Validierung der in dem Klassifizierungsschritt bestimmten Farbkategorie enthält; und
Hinzufügen (713) der abgebildeten Farb- oder Spektralmessungen und der Farbkategorie zu Trainingsdaten, die von dem Klassifikator verwendet werden, nur dann, wenn der Benutzer des Farb- oder Spektralmessgeräts die Farbkategorie validiert (709).

2. Maschinelles Lernverfahren zur Kategorisierung von Farben nach Anspruch 1, wobei das Farb- oder Spektralmessgerät ein Spektralmessgerät ist und die Farb- oder Spektralmessungen Spektralmessungen sind.

3. Maschinelles Lernverfahren zur Kategorisierung von Farben nach Anspruch 2, wobei der Farbraum ein Tristimulus, Lightness (Helligkeit), Chroma (Buntheit) und hue (Farbton), LCh-Farbraum ist.

4. Maschinelles Lernverfahren zur Kategorisierung von Farben nach Anspruch 3, wobei:
die Oberfläche die Oberfläche der Haut ist;
der Schritt des Vornehmens (701) von Spektralmessungen einer Farbe einer Oberfläche mittels eines Spektralmessgeräts umfasst, dass drei Spektralmessungen an der Stirn einer Person, drei Spektralmessungen an der Wange einer Person und drei Spektralmessungen am Hals einer Person vorgenommen werden; und
der Schritt des Abbildens (705) der Spektralmessungen in einen multidimensionalen Merkmalsraum den Schritt des Abbildens von Spektralmessungen in einen 9-dimensionalen Merkmalsraum umfasst.

5. Maschinelles Lernverfahren zur Kategorisierung von Farben nach Anspruch 4, wobei der Klassifikator durch die Anwendung eines K-Nächste-Nachbarn-Algorithmus auf die Trainingsdaten kategorisiert, indem mindestens eines einer Vielzahl von Kategorie-Label-Sets verwendet wird, wobei die Abstandsfunktion des K-Nächste-Nachbarn-Algorithmus eine euklidische Abstandfunktion ist.

6. Maschinelles Lernverfahren zur Kategorisierung von Farben nach Anspruch 5, wobei k = 1 ist.

7. Maschinelles Lernverfahren zur Kategorisierung von Farben nach den Ansprüchen 5 bis 6, wobei die Abstandsfunktion des K-Nächste-Nachbarn-Algorithmus eine standardisierte euklidische Abstandfunktion ist und wobei die Trainingsdaten standardisiert werden, indem jede neu abgebildete Messung und die Trainingsdaten durch den für die entsprechenden Variablen berechneten Standard-Abweichungswert geteilt werden, wobei die Abstandsfunktion des K-Nächste-Nachbarn-Algorithmus eine individuell gewichtete Abstandsfunktion ist, bei der die Gewichtung von 9 Variablen, die zur Messung paarweiser Abstände verwendet werden, für jede Messung unterschiedlich ist.

8. Maschinelles Lernverfahren zur Kategorisierung von Farben nach einem der Ansprüche 5 bis 7, wobei das Verfahren ferner die Schritte umfasst:
Erfassung (706) von Metadaten zu den Spektralmessungen; und
Auswählen (707), zumindest teilweise, auf der Grundlage der Metadaten, von mindestens einem Label-Set, das in dem Klassifizierungsschritt zu verwenden ist, wobei die Metadaten mindestens eines der folgenden Elemente enthalten: einen Zeitstempel, der die Zeit angibt, zu der die Messungen vorgenommen wurden, Details, die sich auf die Identität der Person, den geografischen oder den Einzelhandelsstandort der Person beziehen.

9. Maschinelles Lernverfahren zur Kategorisierung von Farben nach einem der Ansprüche 5 bis 8, wobei jedes Kategorie-Label-Set eine Vielzahl von Labels enthält, die sich auf Farbnuancen von Kosmetikprodukten beziehen, wobei sich die Kategorie-Label-Sets auf verschiedene Kosmetikproduktlinien beziehen und wobei sich die Kategorie-Label-Sets auf verschiedene Kosmetikproduktlinien beziehen, die am geographischen oder am Einzelhandelsstandort der Person erhältlich sind.

10. Computerlesbares Medium, enthaltend Anweisungen, die einen Prozessor bei Verbindung mit einem Farb- oder Spektralmessgerät veranlassen, die Verfahrensschritte gemäß einem der Ansprüche 1 bis 9 auszuführen.

11. Farbkategorisierungssystem, umfassend:
zumindest ein Farb- oder Spektralmessgerät (510) das angeordnet ist, um Farb- oder Spektralmessungen einer Farbe einer Oberfläche von Haut, Haar, Fingernägeln oder einem anderen Körperteil vorzunehmen, wobei das zumindest eine Farb- oder Spektralmessgerät ferner angeordnet ist, um die Farb- oder Spektralmessungen über ein Datenkommunikationsnetz an eine Datenverarbeitungseinheit zu übertragen; und
eine Zentraleinheit (501), die angeordnet ist, um:
die Farb- oder Spektralmessungen zu empfangen;
die Farb- oder Spektralmessungen in einen Farbraum abzubilden;
die Farbraummessungen auf einen multidimensionalen Merkmalsraum abzubilden, um einen multidimensionalen Merkmalsvektor zu erzeugen, der für eine Vielzahl von verschiedenen Messstellen des Körpers repräsentativ ist;
die abgebildeten Farb- oder Spektralmessungen unter Verwendung des erzeugten multidimensionalen Merkmalsvektors und zumindest eines Klassifikators in eine Kategorie einzuordnen;
einen Hinweis auf die Kategorie an das zumindest eine Farb- oder Spektralmessgerät zu senden;
eine Validierungsanfrage zu erzeugen; und
die Validierungsanfrage an das zumindest eine Farb- oder Spektralmessgerät zu senden, wobei das zumindest eine Farb- oder Spektralmessgerät ferner angeordnet ist, um:
die Validierungsanfrage für den Benutzer des Farb- oder Spektralmessgeräts anzuzeigen;
eine Validierungsantwort von dem Benutzer des Farb- oder Spektralmessgeräts anzunehmen;
die Validierungsantwort an die Zentraleinheit zu senden, wobei die Zentraleinheit (501) ferner angeordnet ist, um die abgebildeten Farb- oder Spektralmessungen und Farbkategorien den Trainingsdaten hinzuzufügen, die von dem Klassifikator verwendet werden, wenn eine von dem zumindest einen Spektralmessgerät (510) empfangene Antwort positiv ist.

12. Farbkategorisierungssystem nach Anspruch 11, wobei der Klassifikator angeordnet ist, um durch die Anwendung eines K-Nächste-Nachbarn-Algorithmus auf die Trainingsdaten und durch die Verwendung von zumindest einem einer Vielzahl von Kategorie-Label-Sets zu kategorisieren.

13. Farbkategorisierungssystem nach einem der Ansprüche 11 bis 12, wobei das System ferner umfasst:
eine Metadaten-Erfassungseinrichtung, die angeordnet ist zum Erfassen von Metadaten zu Farb- oder Spektralmessungen; und
eine Auswahleinrichtung, die angeordnet ist, um zumindest teilweise, basierend auf den Metadaten, zumindest ein Label-Set auszuwählen, das von dem Klassifikator zu verwenden ist, wobei die Metadaten folgenden Elemente enthalten: einen Zeitstempel, der die Zeit angibt, zu der die Messungen vorgenommen wurden, Details, die sich auf die Identität der Person, den geografischen oder den Einzelhandelsstandort der Person beziehen.

14. Farbkategorisierungssystem nach einem der Ansprüche 12 bis 13, wobei jedes Kategorie-Label-Set eine Vielzahl von Labels enthält, die sich auf Farbnuancen von Kosmetikprodukten beziehen, wobei sich die Kategorie-Label-Sets auf verschiedene Kosmetikproduktlinien beziehen und wobei sich die Kategorie-Label-Sets auf verschiedene Kosmetikproduktlinien beziehen, die am geographischen oder am Einzelhandelsstandort der Person erhältlich sind.

## Revendications

1. Procédé d'apprentissage automatique de catégorisation de couleurs comprenant les étapes consistant à :
prendre (701) des mesures de couleur ou spectrales d'une couleur d'une surface de la peau, des cheveux, des ongles ou d'une autre partie d'un corps à l'aide d'un dispositif de mesure de couleur ou spectrale ;
mapper les mesures de couleur ou spectrales dans un espace colorimétrique ;
mapper (705) les mesures de l'espace colorimétrique dans un espace de caractéristiques multidimensionnelles pour générer un vecteur de caractéristiques multidimensionnelles représentatif d'une pluralité d'emplacements mesurés différents du corps ;
classer (707) la couleur mappée ou les mesures spectrales dans une catégorie de couleur à l'aide du vecteur de caractéristiques multidimensionnelles généré et d'au moins un classificateur ;
générer une demande de validation (708) pour un utilisateur du dispositif de mesure de couleur ou spectrale, la demande de validation comprenant une demande de validation de la catégorie de couleur déterminée lors de l'étape de classification ; et
ajouter (713) la couleur mappée ou les mesures spectrales et la catégorie de couleur aux données d'apprentissage utilisées par le classificateur uniquement si l'utilisateur du dispositif de mesure de couleur ou spectrale valide (709) la catégorie de couleur.

2. Procédé d'apprentissage automatique de catégorisation de couleurs selon la revendication 1, dans lequel le dispositif de mesure de couleur ou spectrale est un dispositif de mesure spectrale et les mesures de couleur ou spectrales sont des mesures spectrales.

3. Procédé d'apprentissage automatique de catégorisation de couleurs selon la revendication 2, dans lequel l'espace colorimétrique est un espace colorimétrique tristimulus, Luminosité, Chroma et teinte, LCh.

4. Procédé d'apprentissage automatique de catégorisation de couleurs selon la revendication 3, dans lequel :
la surface est la surface de la peau ;
l'étape de prise (701) de mesures spectrales d'une couleur d'une surface à l'aide d'un dispositif de mesure spectrale comprend la prise de trois mesures spectrales du front d'un sujet, de trois mesures spectrales de la joue du sujet et de trois mesures spectrales du cou du sujet ; et
l'étape de mappage (705) des mesures spectrales dans un espace de caractéristiques multidimensionnel comprend l'étape de mappage des mesures spectrales dans un espace de caractéristiques à 9 dimensions.

5. Procédé d'apprentissage automatique de catégorisation de couleurs selon la revendication 4, dans lequel le classificateur catégorise à l'aide d'un algorithme des k plus proches voisins dans les données d'apprentissage en utilisant au moins un parmi une pluralité d'ensembles d'étiquettes de catégorie, dans lequel la fonction de distance de l'algorithme des k plus proches voisins est une fonction de distance euclidienne.

6. Procédé d'apprentissage automatique de catégorisation de couleurs selon la revendication 5, dans lequel k = I

7. Procédé d'apprentissage automatique de catégorisation de couleurs selon les revendications 5 à 6, dans lequel la fonction de distance de l'algorithme des k plus proches voisins est une fonction de distance euclidienne standardisée, et les données d'apprentissage sont standardisées en divisant chaque nouvelle mesure mappées et les données d'apprentissage par le valeurs d'écart type calculées pour les variables correspondantes, la fonction de distance de l'algorithme des k plus proches voisins étant une fonction de distance pondérée personnalisée dans laquelle la pondération des 9 variables utilisées pour mesurer les distances par paires pour chaque mesure diffère.

8. Procédé d'apprentissage automatique de catégorisation de couleurs selon l'une quelconque des revendications 5 à 7, dans lequel le procédé comprend en outre les étapes suivantes :
collecter (706) des métadonnées relatives aux mesures spectrales ; et
sélectionner (707), au moins en partie, sur la base des métadonnées, au moins un ensemble d'étiquettes à utiliser dans l'étape de classification, les métadonnées comprenant au moins l'un des éléments suivants : un horodatage représentant l'heure à laquelle les mesures ont été prises, des détails relatifs à l'identité du sujet, à la localisation géographique ou de vente au détail du sujet.

9. Procédé d'apprentissage automatique de catégorisation de couleurs selon l'une quelconque des revendications 5 à 8, dans lequel chaque ensemble d'étiquettes de catégorie comprend une pluralité d'étiquettes relatives aux nuances de couleurs de produits cosmétiques, dans lequel les ensembles d'étiquettes de catégorie concernent différentes lignes de produits cosmétiques et dans lequel l'étiquette de catégorie les ensembles concernent différentes gammes de produits cosmétiques disponibles dans la zone géographique ou de vente au détail du sujet.

10. Support lisible par ordinateur comprenant des instructions pour amener un processeur, lorsqu'il est connecté à un dispositif de mesure de couleur ou spectrale, à exécuter les étapes du procédé selon l'une quelconque des revendications 1 à 9.

11. Un système de catégorisation des couleurs comprenant :
au moins un dispositif de mesure de couleur ou spectrale (510) agencé pour prendre des mesures de couleur ou spectrales d'une couleur d'une surface de la peau, des cheveux, de l'ongle ou d'une autre partie d'un corps, ledit au moins un dispositif de mesure de couleur ou spectrale étant en outre agencé pour communiquer les mesures de couleur ou spectrales à une unité de traitement de données sur un réseau de communication de données ; et
une unité centrale de traitement (501) agencée pour :
recevoir les mesures de couleur ou spectrales ;
mapper les mesures de couleur ou spectrales dans un espace colorimétrique ;
mapper les mesures de l'espace colorimétrique sur un espace de caractéristiques multidimensionnelles pour générer un vecteur de caractéristiques multidimensionnelles représentatif d'une pluralité d'emplacements mesurés différents du corps ;
classer les mesures de couleur ou spectrales mappées dans une catégorie à l'aide du vecteur de caractéristiques multidimensionnelles généré et d'au moins un classificateur ;
envoyer une indication de catégorie à l'au moins un dispositif de mesure de couleur ou de spectre ;
générer une demande de validation ; et
envoyer la demande de validation à l'au moins un dispositif de mesure de couleur ou spectrale, le au moins un dispositif de mesure de couleur ou spectrale est en outre agencé pour :
afficher la demande de validation à l'utilisateur du dispositif de mesure couleur ou spectrale ;
accepter une réponse de validation de la part de l'utilisateur du dispositif de mesure couleur ou spectrale ;
envoyer la réponse de validation à l'unité centrale de traitement, l'unité centrale de traitement (501) étant en outre conçue pour ajouter la couleur mappée ou les mesures spectrales et la catégorie de couleur aux données d'apprentissage utilisées par le classificateur si une réponse de validation reçue du au moins un spectre le dispositif de mesure (510) est positif.

12. Système de catégorisation de couleurs selon la revendication 11, dans lequel le classificateur est agencé pour catégoriser en utilisant un algorithme des k plus proches voisins sur les données d'apprentissage et en utilisant au moins un parmi une pluralité d'ensembles d'étiquettes de catégorie.

13. Système de catégorisation de couleurs selon l'une quelconque des revendications 11 à 12, dans lequel le système comprend en outre :
des moyens de collecte de métadonnées agencés pour collecter des métadonnées relatives aux mesures de couleur ou spectrales ; et
des moyens de sélection conçus pour sélectionner, au moins en partie, sur la base des métadonnées, au moins un ensemble d'étiquettes à utiliser par le classificateur, les métadonnées comprenant au moins l'un des éléments suivants : un horodatage représentant l'heure à laquelle la mesure a été effectuée, des détails relatifs à l'identité du sujet, à la localisation géographique ou de vente au détail du sujet.

14. Système de catégorisation de couleurs selon l'une quelconque des revendications 12 à 13, dans lequel chaque ensemble d'étiquettes de catégorie comprend une pluralité d'étiquettes relatives aux nuances de couleurs de produits cosmétiques, dans lequel les ensembles d'étiquettes de catégorie concernent différentes lignes de produits cosmétiques, dans lequel les ensembles d'étiquettes de catégorie concernent différentes gammes de produits cosmétiques disponibles dans la zone géographique ou de vente au détail du sujet.
